(19)

**Europäisches Patentamt**
**European Patent Office**
**Office européen des brevets**

(11) **EP 4 486 211 B1**

(12) **EUROPEAN PATENT SPECIFICATION**

(45) Date of publication and mention
of the grant of the patent:
**03.12.2025 Bulletin 2025/49**

(21) Application number: **22713452.5**

(22) Date of filing: **03.03.2022**

(51) International Patent Classification (IPC):
*A61B 6/50* (2024.01)      *A61B 6/00* (2024.01)
*G16H 30/40* (2018.01)      *G16H 50/50* (2018.01)

(52) Cooperative Patent Classification (CPC):
**G16H 50/50; A61B 6/503; A61B 6/504;**
**A61B 6/507; A61B 6/5217; G16H 30/40**

(86) International application number:
**PCT/IB2022/051883**

(87) International publication number:
**WO 2023/166333 (07.09.2023 Gazette 2023/36)**

(54) **ASSESSMENT OF MYOCARDIAL PERFUSION USING NON-INVASIVE MEASUREMENTS AND POROMECHANICS**

BEURTEILUNG DER MYOKARDIALEN PERFUSION UNTER VERWENDUNG NICHTINVASIVER MESSUNGEN UND POROMECHANIK

ÉVALUATION DE PERFUSION MYOCARDIQUE À L'AIDE DE MESURES NON INVASIVES ET DE POROMÉTRIE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**08.01.2025 Bulletin 2025/02**

(73) Proprietor: **HEMOLENS DIAGNOSTICS SPÓLKA Z**
**OGRANICZONA**
**ODPOWIEDZIALNOSCIA**
**54-202 Wroclaw (PL)**

(72) Inventor: **MIROTA, Kryspin**
**52-200 Wroclaw (PL)**

(74) Representative: **JWP Patent & Trademark**
**Attorneys**
**ul. Minska 75**
**03-828 Warszawa (PL)**

(56) References cited:
**US-A1- 2019 183 579**

• **LEE JACK ET AL: "In silicocoronary wave intensity analysis: application of an integrated one-dimensional and poromechanical model of cardiac perfusion", BIOMECHANICS AND MODELING IN MECHANOBIOLOGY, SPRINGER BERLIN HEIDELBERG, BERLIN/HEIDELBERG, vol. 15, no. 6, 23 March 2016 (2016-03-23), pages 1535 - 1555, XP036097907, ISSN: 1617-7959, [retrieved on 20160323], DOI: 10.1007/ S10237-016-0782-5**

• **GOVINDARAJU KALIMUTHU ET AL: "Effect of porous media of the stenosed artery wall to the coronary physiological diagnostic parameter: A computational fluid dynamic analysis", ATHEROSCLEROSIS, ELSEVIER, AMSTERDAM, NL, vol. 233, no. 2, 31 January 2014 (2014-01-31), pages 630 - 635, XP028634454, ISSN: 0021-9150, DOI: 10.1016/J.ATHEROSCLEROSIS.2014.01.043**

• **KHAKPOUR ET AL: "Critical assessment of arterial transport models", INTERNATIONAL JOURNAL OF HEAT AND MASS TRANSFER, ELSEVIER, AMSTERDAM, NL, vol. 51, no. 3-4, 28 June 2007 (2007-06-28), pages 807 - 822, XP022411847, ISSN: 0017-9310, DOI: 10.1016/ J.IJHEATMASSTRANSFER.2007.04.021**

**(Cont. next page)**

• SOULIS ET AL: "Non-Newtonian models for molecular viscosity and wall shear stress in a 3D reconstructed human left coronary artery", MEDICAL ENGINEERING & PHYSICS, BUTTERWORTH-HEINEMANN, GB, vol. 30, no. 1, 31 December 2007 (2007-12-31), pages 9 - 19, XP022422780, ISSN: 1350-4533, DOI: 10.1016/J.MEDENGPHY.2007.02.001

• QOHAR ULIN NUHA ABDUL: "CFD Generated Tracer Indicator Flow Assessment using Perfusion MRI Analysis", 2020 IEEE-EMBS CONFERENCE ON BIOMEDICAL ENGINEERING AND SCIENCES (IECBES), IEEE, 1 March 2021 (2021-03-01), pages 51 - 56, XP033902621, DOI: 10.1109/IECBES48179.2021.9398769

**Description**

FILED OF THE INVENTION

The invention pertains to myocardial perfusion imaging (MPI) and poromechnics. In particular, the invention involves the use of non-invasive measurements (for example, computed angiography or a nuclear magnetic resonance) to build a model comprising a porous domain of a human heart to be used in computational fluid dynamics. The results of the invention allow to diagnose the human heart. For example, to diagnose a coronary heart disease.

BACKGROUND

One of the major challenges for the application of perfusion assessment methods in medical diagnosis is a coronary heart disease (CHD). According to the National Center for Health Statistics, 5,6% of U.S. adults had a coronary heart disease in 2018. Prevalence increased with age and almost 24% of people at the age of 75 and over had a CHD. The disease affected men more often than women - 7.4% vs 4.1% of all U.S. adults (according to Villarroel MA, Blackwell DL, Jen A (2019) Tables of Summary Health Statistics for U.S. Adults: 2018 National Health Interview Survey, National Center for Health Statistics. Accessed 15 Dec 2021). In 2018, a CHD caused 365 744 deaths in the U.S. which accounts for almost 13% of all deaths therein (see Centers for Disease Control and Prevention, National Center for Health Statistics, National Vital Statistics System: public use data file documentation: mortality multiple cause-of-death micro-data files. Accessed 15 Dec 2021). In 2017, 623 276 people died from an ischemic heart disease in the European Union which accounts for almost 12% of all deaths therein in 2018 (see Eurostat (2021) Causes of death - deaths by country of residence and occurrence [HL_TH_CD_ARO]. Accessed 15 Dec 2021).

A heart is the center of the circulatory system. A human heart beats about 100 000 times per day which sums to about 3 billion beats in a lifetime. Pumping of a heart and a heartbeat are caused by alternating contractions and relaxations of the myocardium. This pumping ensures continuity of blood movement in the vessels. Thus, a heart is responsible for fulfilling various functions in a human body: provision of oxygen and nutrients, removal of metabolites, protection from foreign bodies, toxic substances and against exsanguination. Moreover, a heart is responsible for maintenance of constant physical and chemical conditions in a human body, especially temperature and pH (according to Barrett KE et all (2019) Ganong's Review of Medical Physiology, 26th Edition, McGraw-Hill Education). Indeed, the modern medical diagnostics offer a wide spectrum of methods and parameters for a functional assessment of a heart. Despite this, like a hundred and more years ago, the key role in medical diagnostics is played by a parameter that describes the amount of blood pumped within a certain time interval, usually within a single cycle or a minute. As long as this parameter is maintained at an adequate level, the prerequisite for other organs to maintain their functions is fulfilled. The awareness of this principle and attempts to evaluate this parameter can be found in very early experimental works on physiology. Already in 1761, Albrecht von Haller was studying a pulmonary circulation by an injection of a colored liquid into the vena cava of a fresh animal corpses (see Haller A (1761) Elementa physiologiae corporis humani, Francisci Grasser & Sociorum, Lausanne). The turning point and the foundation of the modern methods of flow diagnostics with indicator-dilution techniques, i.e., the techniques commonly used in modern times to assess perfusion, was the research done by George Stewart (see Argueta EE, Paniagua D (2019) Thermodilution cardiac output, Cardiology in Review, 27(3), p. 138-144) and then, less than a century later, the work done by Kenneth Zierler (see Meier P, Zierler KL (1954) On the theory of the indicator-dilution method for measurement of blood flow and volume, The Journal of Applied Physiology, 6(12), p. 731-744, Zierler K (2000) Indicator dilution methods for measuring blood flow, volume, and other properties of biological systems: a brief history and memoir, Annals of Biomedical Engineering, 28(8), p. 836-848).

A myocardial prefusion, i.e., a blood flow through a specific mass of a tissue, is the basic physiological parameter determined on the basis of cardiological image data. Perfusion imaging - including myocardial perfusion imaging (MPI) - involves injecting a contrast into the blood and recording distribution of changes in its concentration in the cardiovascular system over time. A key issue in a perfusion analysis is the calculation of so-called perfusion parameters or perfusion metrics such as myocardial blood flow *MBF,* myocardial blood volume *MBV,* mean transit time *MTT* and time to peak *TTP* (following Taylor SH (1966) Measurement of the cardiac output in man, Proc R Soc Med, 59 Suppl(Suppl 1), p. 35-53, Axel L (1980) Cerebral blood flow determination by rapid-sequence computed tomography: theoretical analysis, Radiology, 137(3), p. 679-86, Lee T-Y (2002) Functional CT: Physiological models, Trends Biotechnol, 20, p. S3-S10, Tomandl BF et al. (2003) Comprehensive imaging of ischemic stroke with multisection CT, Radiographics, 23(3), p. 565-92, Konstas AA et al. (2009) Theoretic basis and technical implementations of CT perfusion in acute ischemic stroke. Part 1: Theoretic basis, AJNR Am J Neuroradiol, 30(4), p. 662-8). Let's assume a certain control volume of interest (VOI) with a uniquely defined inlet and an outlet. If a certain amount ($m$) of a contrast agent was introduce at the initial moment $t_0 = 0$, and a sample was taken on the outlet side, after the time $\Delta t_i$, in which the concentration $c(t_i)$, $t_i \in \Delta t_i$ of the contrast agent was measured, then the mass of the contrast agent that left VOI is expressed by

$$\Delta m(t_i) = c(t_i)\Delta V(t_i)\,, \tag{1}$$

where $\Delta V(t_i) = q(t_i) \cdot \Delta t_i$ is a volume of blood that flowed through an outlet section. The total mass of the contrast agent that will reach the outlet, after sampling at the outlet and determining the concentration for a sufficiently long time, is expressed by

$$m = \sum_i \Delta m(t_i) = \sum_i c(t_i)q(t_i) \cdot \Delta t_i\,. \tag{2}$$

If samples were taken frequently enough than we have

$$m = \int_0^\infty c(t)q(t)dt\,. \tag{3}$$

Hence, based on the mean value theorem we can arrive at

$$m = q(t_i)\int_0^\infty c(t)dt = \bar{Q}\int_0^\infty c(t)dt,\ t_i \in (0,\infty)\,, \tag{4}$$

where $\overline{Q}$ is the mean value of a blood flow that perfuses through VOI. Referring to a myocardial perfusion and a cardiac output (CO), we obtain the following relationship

$$CO = \frac{m}{\int_0^\infty c(t)dt} = \frac{m}{AUC}\,, \tag{5}$$

that describes the volume (e.g., in liters) of blood being pumped by a heart per unit time (e.g., per minute) as the amount of contrast agent at the inlet divided by the area under concentration curve (AUC) recorded at the outlet.

If we analyze an *in vivo* recording of the arterial input function (AIF) and the time attenuation curve (TAC): a change of contrast agent quantity that occurs in a certain time interval is directly proportional to the difference between inflow and outflow quantity. This can be expressed by

$$V_t \frac{dc_t(t)}{dt} = Q_t \cdot \big(c_a(t) - c_v(t)\big)\,, \tag{17}$$

which determines the balance of the mass flux of a contrast agent in the control volume $V_t$ of the tissue, where the average flow is $Q_t$. The concentration $c(t)$ in the region monitored by the arterial input AIF curve was denoted as $c_a(t)$ and the tissue attenuation TAC curve as $c_t(t)$, and the concentration in venous outflow as $c_v(t)$. Given that relative unit values rather than absolute values are used in the perfusion analysis, it is appropriate to express the mean flow $Q$ accordingly by the mean volume of flowing blood (MBF - myocardial blood flow) in a bulk of a tissue:

$$MBF = \frac{Q_t}{\rho_t V_t}\ \ \to\ \ \frac{Q_t}{V_t} = \rho_t MBF\,, \tag{18}$$

where $\rho_t$ is tissue density. Thus, the output balance equation takes the form of

$$\frac{dc_t(t)}{dt} = \rho_t MBF \cdot \big(c_a(t) - c_v(t)\big)\,. \tag{19}$$

When analyzing the shape of TAC, it can be noticed that in the initial phase there is a gradual accumulation of the contrast agent in the control volume and the outflow is negligible, and the concentration curve has a strictly increasing character. For this phase, assuming that the analysis is only limited to the part where the outflow is negligibly small, i.e., $c_v(t) \approx 0$, the balance equation takes the form of

$$\frac{dc_t(t)}{dt} = \rho_t MBF \cdot c_a(t)\,, \tag{20}$$

and this assumption can be additionally strengthened by limiting our considerations only to the maximum values

$$\max\left(\frac{dc_t(t)}{dt}\right) = \rho_t MBF \cdot \max\big(c_a(t)\big) \ . \tag{21}$$

So, if we want to assess perfusion (MBF - myocardial blood flow) in a practical way, we can use the maximum slope method. Then MBF is expressed as the ratio of the maximum slope of TAC for the accumulation phase to the maximum value recorded on AIF, multiplied by the reciprocal of the tissue density $\rho_t$

$$MBF = \frac{1}{\rho_t} \cdot \frac{\max\left(\frac{d}{dt}TAC\right)}{\max(AIF)} \ . \tag{22}$$

By the same reasoning and by starting from the mass balance and not its flux as in equation (17), we obtain the relation for the unit volume of the vascular bed, which is called the myocardial blood volume (MBV)

$$MBV = \frac{1}{\rho_t} \cdot \frac{\max(TAC)}{\max(AIF)} \ . \tag{23}$$

Calculation of the perfusion time parameters, e.g., mean MTT and max TTP, is a purely arithmetic operation

$$MBF = \frac{MBV}{MTT} \quad \rightarrow \quad MTT = \frac{MBV}{MBF} \ . \tag{24}$$

TTP is simply a coordinate on the time axis for which the following relation

$$TAC(TTP) = \max(TAC) \tag{25}$$

is satisfied. The maximum slope method is a method with extremely high efficiency and moderate hardware requirements. It is possible to apply it effectively even to very large size data sets. This is of considerable importance for analyzing of computed tomography (CT) perfusion imaging data.

The field of noninvasive clinical cardiac imaging is rapidly evolving. The evolution is focused on quantitative perfusion assessment technologies which can be used for assessing a coronary flow and a myocardial ischaemia. The main methods for myocardial perfusion assessment are the single photon emission computed tomography (SPECT), the positron emission tomography (PET) and the stress dynamic computed tomography perfusion (CTP) (see AlJaroudi WA, Hage FG (2020) Review of cardiovascular imaging in the Journal of Nuclear Cardiology 2019: Positron emission tomography, computed tomography and magnetic resonance, J Nucl Cardiol, 27(3), p. 921-930).

For decades, SPECT has been an established method for clinical cardiovascular imaging. The evaluation of a myocardial ischaemia extent based on SPECT, taken together with measurements of a left ventricular function and volumes, is considered to be an approved method for diagnosis of a myocardial ischaemia. The main goals of the SPECT technical evolution were maximizing sensitivity, reducing of acquisition time and reducing amounts of injected radiopharmaceuticals. A SPECT temporal resolution (which is a frame acquisition time) is equal to 10s and a voxel size is $10\times10\times10$ mm$^3$. In addition, there was the problem of increasing BMI (body mass index) in patient population. The solution to the problem was cadmium-zinc-telluride solid-state detector technology (CZT) which provided an increased sensitivity, an increased spatial and energy resolution and a decreased acquisition time and with smaller doses of radiopharmaceuticals. CZT SPECT enabled a dynamic imaging and kinetics assessment of perfusion tracers in blood and in the myocardium. With this information, a compartmental modelling and delineation of absolute MBF and a coronary flow reserve are possible (see Dewey M (2020) Clinical quantitative cardiac imaging for the assessment of myocardial ischaemia, Nature Reviews Cardiology, 17(7), p. 427-450).

Another technique for confirming or excluding ischaemia is the PET. PET allows for an accurate *in vivo* measurement of concentrations of radiopharmaceuticals. A PET temporal resolution (which is a frame acquisition time) is in the range of 1 - 5 s and a voxel size is $4\times4\times4$ mm$^3$. Different markers are used in examination, but $^{15}$O-water is considered to be a clinical gold standard. Modelling of $^{15}$O-water kinetics is well-established and tracked concentration thereof is directly proportional to measured image signal. Because of short half-lives of tracers, rest-stress protocols can be completed in 30 min, therefore a total radiation dose is lower than that in SPECT. Images of perfusion defects are sometimes grainy and difficult to interpret. MBF values can be provided by modelling tracer kinetics and correction for limited extraction. Recent advances in PET allowed for computation of parametric images showing voxel-level MBF and facilitated $^{15}$O-water PET use in the clinical environment (see Dewey M (2020) Clinical quantitative cardiac imaging for the assessment of myocardial ischaemia, Nature Reviews Cardiology, 17(7), p. 427-450).

The last of these three techniques is CTP. CTP presently has two approaches: static and dynamic. The assessment of myocardial enhancement acquired at a single time point during the first-pass of a contrast sample is referred to as a static CTP imaging. The dynamic CTP, on the other hand, assesses a myocardial perfusion by measuring myocardial enhancement at numerous time intervals during the initial pass of a contrast sample, which is bolus timing resilient. The dynamic CTP allows for a fully quantitative examination of a myocardial perfusion. Due to the recent advancements in a computed tomography (CT) technology, the dynamic CTP of the whole myocardium may now be conducted with a significantly less radiation exposure (see Kitagawa K (2018) Dynamic CT perfusion imaging: state of the art, Cardiovascular Imaging Asia, 2(2), p. 38-48).

All the above-mentioned methods have both advantages and disadvantages. In terms of spatial and temporal resolutions, a quality of an image and an accuracy of diagnosis, PET outperforms SPECT. PET can be also performed in patients with implantable cardioverter defibrillators and in those with pacemakers. However, PET involves high costs, a high radiation exposure and is not so widely available. SPECT is more suitable for a clinical routine, because of radionuclides being less expensive, easier to prepare and having longer half-lives. In addition, SPECT performs better in detection of an ischaemia due to its high sensitivity and specificity. SPECT allows for the assessment of a left ventricular function. The limitations of SPECT are high costs, time consumption, a radiation exposure, limited anatomical information because of a poor spatial resolution, attenuation artefacts producing false positive results (especially in obese patients), possibility of underestimating the actual extent of an ischaemia in patients with a multivessel disease. Unlike other mentioned methods, a CTP imaging is the only non-invasive method for identifying functional significance of coronary stenosis and quantifying it. A single CTP examination provides an integrated anatomic and a functional evaluation. Moreover, it is very fast and a widely available method with a high sensitivity and specificity. CTP allows for detection of smaller perfusion defects due to a high spatial resolution (approximately 0.3 mm) thereof. However, this method suffers from radiation exposure and motion artefacts originating from breathing (breath artefacts), beam hardening and high heart rate artifacts (following Xu C, Yi Y & Wang Y (2020) Clinical Applications of CT Myocardial Perfusion Imaging, Cardiovascular Imaging Asia, 4(4), p. 86-90, Seitun S (2018) CT myocardial perfusion imaging: a new frontier in cardiac imaging, BioMed research international, 2018).

A progress in a computed tomography technology in recent years has allowed for the clinical application of a myocardial perfusion imaging by the computed tomography (CTP). In line with PET and the magnetic resonance imagining (MRI), CTP may provide qualitative or quantitative perfusion data. An assessment of a myocardial perfusion by CT can be achieved through a static or a dynamic scan acquisition. Although both types of acquisition have proven to be clinically applicable, important barriers must be overcome before they will be used routinely. For example, a success of a perfusion assessment using the static CTP is highly dependent on a contrast material bolus timing and motion. In contrast, the dynamic CTP of a myocardial perfusion assessment allows for a fully quantitative analysis of a myocardial perfusion. A myocardium is enhanced at a plurality of time points of first-pass of a contrast agent, in that way a myocardium perfusion is assessed. An assessment of a myocardial perfusion may also be affected by performing stress CTP studies with and without a beta-blockade (see Seitun S (2016) Stress computed tomography myocardial perfusion imaging: a new topic in cardiology, Revista Española de Cardiologia (English Edition), 69(2), p. 188-200).

During perfusion examination, difficulties can arise in establishing clear cut-off MBF values. Among researchers, the cut-off values of the MBF derived from CT vary between 75 and 164 mL/100 mL/min. Differences in a study design, a patient coronary risk profile, sample sizes, an FFR threshold value, and prevalence of a CAD may be the reason for this wide range of cut-off MBF values (see Kitagawa K (2018) Dynamic CT perfusion imaging: state of the art, Cardiovascular Imaging Asia, 2(2), p. 38-48). When MBF is uniformly high (i.e., >110 mL/100 mL/min for the dual-source CT and a maximum slope technique), a stress myocardial perfusion can be regarded to be normal. A low MBF (75 mL/100 mL/min) is uncommon, however it is most likely related to a lack of or to an inadequate hyperemic response to vasodilators, which might make ischemia identification difficult. Once sufficient hyperemia has been proven by a high MBF in a remote myocardium, a hemodynamic significance of a coronary artery stenosis can be determined using a relative MBF value in the vascular region. Because of normal MBF patient-to-patient variations, a generally applicable absolute MBF threshold for a myocardial ischemia may not exist (see Kitagawa K (2018) Dynamic CT perfusion imaging: State of the art, Cardiovasc Imaging Asia, 2(2), p. 38-48).

With a CTP imaging, we can assess with an unprecedented spatial resolution what is happening *in vivo.* This contributes to a better understanding of a myocardial perfusion at a microvascular level. In recent years, the development in this topic have led to a reduction of: a scan time, motion artifacts, use of a contrast agent and a radiation dose exposure. This has also led to a significant improvement in a spatial and a temporal resolution. However, all the above aspects still remain problematic and have their limitations.

For MPI, as mentioned above, we face the problem of artefacts that impede an accurate evaluation of a myocardial perfusion, namely artefacts that are caused by the motion of a heart. The artifacts may lead to the appearance of hypoperfused areas that may mistakenly resemble myocardial ischaemia. In that scenario, we are facing the problem of identifying what defects are relevant for perfusion and which are false positive findings. The above-mentioned motion artefacts can arise for several reasons and, for example, by a patient movement during the scan procedure or by a cardiac motion because of high or irregular heart rates. Examination of different cardiac phases is a way to reduce the impact of motion artefacts in MPI scans. Specifically, real (or true) perfusion defects can be observed in all phases of the cardiac cycle, whereas a motion artefact is visible only in a few cardiac phases. In addition, problems in a temporal resolution can be caused by the occurrence of motion artifacts, especially in patients with a high heart rate and a high heart rate variability. A sufficiently high temporal resolution as well as a sufficiently high spatial resolution are required during a dynamic CTP imaging of a heart rate at stress to image the entire myocardium which is "working" at a high rate (following Seitun S (2016) Stress computed tomography myocardial perfusion imaging: a new topic in cardiology, Revista Española de Cardiologia (English Edition), 69(2), p. 188-200, Seitun S (2018) CT myocardial perfusion imaging: a new frontier in cardiac imaging, BioMed research international, Volume 2018, Article ID 7295460, 21 pages). Cardiac patients, due to their medical conditions, may undergo multiple imaging procedures which increases their radiation exposure above what is expected for a lifetime. For example, the dynamic CTP is associated with high radiation doses, because this imaging technique requires multiple acquisitions to generate the TACs. During the static CTP, an average radiation patient exposure is in the range of 1.9 - 15.7 mSv with an average value of 5.93 mSv. For the dynamic CTP imaging, effective dose values are in the range of 3.8 - 12.8 mSv with an average radiation dose of 9.23 mSv. A CT-based dynamic myocardial perfusion imaging requires a relatively high radiation exposure. Various techniques are used to reduce radiation doses, for example, a low tube voltage is set during the dynamic CTP for patients with normal body mass indexes. In this way, the radiation dose can be reduced by 40% while maintaining image quality and a good MBF assessment. However, improvement of CTP imaging procedures is still needed. The improvement is to eliminate unnecessary radiation exposure and risks associated with this procedure (see Danad I (2016) Static and dynamic assessment of myocardial perfusion by computed tomography, European Heart Journal-Cardiovascular Imaging, 17(8), p. 836-844).

Methods based on the indicator-dilution flow analysis have been developed for a long time and are not only limited to application in typical medicine. There is a very wide range of applications of these method in hydrology and hydrogeology as well as in chemical and process engineering. In particular, the methods were used in cases of moving rocks or debris in a stream bed, when a stream cross-section was indeterminant or flow was physically inaccessible or unsafe (see Florkowski T, Davis TG (1969) The measurement of high discharges in turbulent rivers using tritium tracer. Journal of Hydrology, 8(3), p. 249-264, Kilpatrick FA, Cobb ED (1982) Measurement of discharge using tracers, U.S. Department of the Interior, U.S. Geological Survey, Evans GV (1983) Tracer techniques in hydrology, The International Journal of Applied Radiation and Isotopes, 34(1), p. 451-475). One should note a change of terminology between engineering and medicine. In particular, two equivalent engineering terms "dye-dilution" and "tracer-dilution" are replaced by one medical term "indicator-dilution". Accordingly, a cardiac output is measured in physiology and a flow discharge is measured in hydrotechnics. The latter was described by Charles Allen in 1927. Charles Allen named the method as the "salt-velocity" method (see Allen CM (1927) Hydraulic - turbine tests by the Allen method, Power Plant Engineering, 31(10), p. 549-551). Tetrach of Trachonitis (A.D. 37) used chaff to trace underground streams of the likely source of the Jordan (see page 247 in Assaad FA, LaMoreaux PE, Hughes T (2012) Field methods for geologists and hydrogeologists, Springer-Verlag Berlin and Heidelberg GmbH & Co. KG). In accordance with circulatory physiology, a mean transit time (MMT or an equivalently tissue transit time - TTT) is introduced. This results from the fact that a highly important parameter is residence time or residence time distribution in chemical engineering and reactor design (following Wingard LB, et al. (1972) Concepts of residence time distribution applied to the indicator-dilution method, J Appl Physiol, 33(2), p. 264-275). Nowadays, the methods based on indicator-dilution flow analysis (also classified as indicator-dilution class methods) are partially or fully substituted - mostly - by computational fluid dynamics based on numerical simulations (following Chen K (2018) CFD simulation of particle residence time distribution in industrial scale horizontal fluidized bed, Powder Technology, 345(1), p. 129-139, Walker P Sheikholeslami R (2003) Assessment of the effect of velocity and residence time in CaSO4 precipitating flow reaction, Chemical Engineering Science, 58(16), p. 3807-3816).

This review of prior art developments is underlying that there is a need for a reliable method that will allow to diagnose patients without the above-mentioned risks and problems. The present invention addresses this need.

US 2019/183579 A1 relates to modeling of hemodynamic parameters in coronary arteries while the coronary arteries are including arterial branches. This document discloses gathering non-invasively data which can include myocardium. There is no anatomical model of a myocardium disclosed therein. The inventive solution requires also continuous arterial pressure data that are gathered non-invasively and which are used for determining boundary conditions for CFD simulations. The CFD simulations made using the anatomical model of only coronary arteries with boundary conditions determined using continuous arterial pressure data are allowing to calculate hemodynamic parameters of arteries. The disclosed hemodynamic parameters are limited to those specific for arteries and there is no disclosure of perfusion parameters or parameters of myocardium.

A scientific paper "Effect of porous media of the stenosed artery wall to the coronary physiological diagnostic parameter: A computational fluid dynamic analysis" by G. Kalimuthu et al, Atherosclerosis, Elsevier, Amsterdam, NL, vol.

233, no. 2, 31 January 2014, p. 630-635 is a about a functional assessment of coronary arteries. This paper assumes that the walls of arteries are porous and describes a flow in arteries. The document is silent about flow in a myocardium.

A scientific paper "Critical assessment of arterial transport models" by Khakpour et al, International Journal of Heat and Mass Transfer, Elsevier, Amsterdam, NL, vol. 51, no. 3-4, 28 June 2007, p. 807-822 is relating to a diffusion through an artery wall. There is no disclosure of flow in a myocardium. The artery wall is treated as a porous medium.

A scientific paper "Non-Newtonian models for molecular viscosity and wall shear stress in a 3D reconstructed human left coronary artery" by Soulis et al, Medical Engineering & Physics, Butterworth-Heinemann, GB, vol. 30, no. 1, 31 December 2007, p. 9-19 relates to invasive measurements that are to obtain a viscosity parameters of blood flow in arteries.

A scientific paper "CFD Generated Tracer Indicator Flow Assessment using Perfusion MRI Analysis" 2020 IEEE-EMBS Conference on Biomedical Engineering and Science (IECBES), IEEE, 1 March 2021, p. 51-56 relates to modeling on arterial, venous systems, a capillary bed and connection between them.

A scientific paper "In silico coronary wave intensity analysis: application of an integrated one-dimensional and poromechanical model of cardiac perfusion" Biomech Model Mechanobiol, vol. 15, no. 6, p. 1535-1555, DOI 10.1007/s10237-016-0782-5 is presenting invasive wave intensity studies on a pig. Those studies involve modeling of overlapping porous and fluid domains with windkessel-type systemic boundary conditions.

BRIEF DESCRIPTION OF THE INVENTION

The forthcoming description is to better understand the principle and benefits of the invention claimed in the appended claims. Therefore, it is not meant to be limiting in any sense. In particular, certain explanations to the meaning or to the function cannot be understood as limiting or exhaustive. They are provided to facilitate understanding and cannot replace the full understanding provided by a common general knowledge of a skilled person in this field.

The present invention redefines the myocardial perfusion imaging (MPI) paradigm. Its core and one of the biggest improvements over the paradigm is replacement of an *in vivo* imaging phase (usually realized with PET, SPECT, or MR scanners) with an *in silico* "imaging" phase while providing medically relevant and consistent results. The central element of the invention is a model of an arterial and myocardial blood flow solved using numerical fluid dynamic techniques. This way, all the insufficiencies of the known *in vivo* approach, including all the risks to the overall health of patients undergoing the procedure, are eliminated. In addition to that, a practically unlimited spatial-temporal resolution is achieved. Moreover, the result of the invention is completely free of motion artifacts and the patient is not additionally exposed to radiation. At the same time, the cost of performing this kind of *in silico* analysis is significantly lower.

Within the scope of this disclosure, the term "tracker" can be used interchangeably with the term "contrast". The term "porous" should be understood as including or being equal to the term "saturated porous".

In one aspect, the invention pertains to a method for assessment of a myocardial perfusion using a non-invasive measurement and poromechnics for a human patient. In particular, poromechnics involve computational fluid dynamics. The method is a computer-implemented method.

The method comprises generating an anatomical geometry model ($\overline{\Omega}$) of a myocardium connected with a plurality of coronary vessels using patient-specific anatomical structure data of the human patient. The patient-specific anatomical structure data of the human patient are obtained from at least one said non-invasive measurement. The term "non-invasive measurement" means a measurement that does not involve any type of surgery and/or significant health risks. In some embodiments, said measurement does not include insertion of any probe into a patient's body. In general, the term "non-invasively" means not involving surgery and/or significant health risks. In certain embodiments, "non-invasively" does not include insertion of any probe into a patient's body.

The anatomical geometry model ($\overline{\Omega}$) of a myocardium connected with a plurality of coronary vessels comprises a fluid domain ($\Omega_f$) and a porous domain ($\Omega_p$) and an interface ($\Gamma$) between the fluid domain ($\Omega_f$) and the porous domain ($\Omega_p$). The anatomical geometry model ($\overline{\Omega}$) of a myocardium connected with a plurality of coronary vessels satisfies the following equations: $\overline{\Omega} = \overline{\Omega}_f \cup \overline{\Omega}_p$ and $\Omega_f \cap \Omega_p = \varnothing$. This means that the fluid domain ($\Omega_f$) and the porous domain ($\Omega_p$) do not overlap. A myocardium connected with a plurality of coronary vessels is described as a composition of $\overline{\Omega}_f$ and $\overline{\Omega}_p$. $\overline{\Omega}_f$ comprises or consists of the fluid domain ($\Omega_f$) and a boundary ($\Gamma_f$) of the fluid domain ($\Omega_f$) and $\overline{\Omega}_p$ comprises or consists of the porous domain ($\Omega_p$) with a boundary ($\Gamma_p$) of the porous domain ($\Omega_p$). Furthermore, the interface ($\Gamma$) between the fluid domain ($\Omega_f$) and the porous domain ($\Omega_p$) satisfies the following equation: $\Gamma = \overline{\Omega}_f \cap \overline{\Omega}_p \neq \varnothing$. This means that the fluid domain ($\Omega_f$) and the porous domain ($\Omega_p$) are connected to each other with the use of the interface ($\Gamma$). At the same time, the interface ($\Gamma$) cannot be "empty". Furthermore, the interface ($\Gamma$) comprises or consists of the boundary ($\Gamma_p$) of the porous domain ($\Omega_p$) and the boundary ($\Gamma_f$) of the fluid domain ($\Omega_f$). The porous domain ($\Omega_p$) can be used to describe flow in a myocardium tissue. The results of the method allow for diagnosis of a heart condition. In particular, the method allows to diagnose a coronary artery disease or a heart failure.

In addition to above-mentioned benefits and the new way for approaching myocardial perfusion imaging (MPI), the use of the fluid domain and the porous domain allows for simplification of description of a myocardium connected with a plurality of coronary vessels. This allows for faster calculations and for arriving at perfusion metrics faster. At the same time, it should be emphasized that building an accurate complex model of a myocardium connected with a plurality of coronary vessels without the use of the fluid and porous domain may not always be possible.

In embodiments, the patient-specific anatomical structure data are obtained via at last one of a computed tomography angiography (CTA), a nuclear magnetic resonance (NMR) and/or a computed angiographic. This anatomical geometry model can be used in computational fluid dynamics to calculate perfusion metrics.

In an embodiment, the patient-specific anatomical structure data comprises images stored in a DICOM format. This allows for reconstruction of time attenuation curve (TAC) and arterial input function (AIF) curves for a number of time points.

The step of generating of the anatomical geometry model ($\overline{\Omega}$) of a heart myocardium connected with a plurality of coronary vessels may comprise segmentation of images comprised in the patient-specific anatomical structure data to arrive at a surface model of the arteries and muscles of a patient's heart. Furthermore, the step of generating of the anatomical geometry model ($\overline{\Omega}$) of a heart myocardium connected with a plurality of coronary vessels may comprise a step in which the surface model is discretized through an unstructured volumetric mesh.

The method further comprises determining at least one patient-specific boundary condition and selecting at least one physical parameter for describing blood as a flow medium using patient-specific demographic and general medical data. The boundary condition and the physical parameter are to be used in computational fluid dynamics to, for example, calculate perfusion metrics. The term "patient-specific" means specific for patient and not e.g., data or parameters for a population of patients. The use of patient-specific data allows for obtaining results that are medically relevant for a particular patient and not mean results for a population of patients.

In one embodiment, the patient-specific demographic and general medical data comprise at least one of patient's age, sex, height, weight, systemic blood pressure, blood test results and/or information on the current patient's pharmacological therapy. The current patient's pharmacological therapy may be selected from, but not limited to, a beta-adrenergic blocking agent, an angiotensin-converting-enzyme inhibitor and/or an antiarrhythmic agent.

In an embodiment, the physical parameter used to for describe blood as a flow medium is selected from the group comprising density and a dynamic viscosity coefficient.

The method further comprises simulating a blood flow in the anatomical geometry model ($\overline{\Omega}$) of a heart myocardium connected with a plurality of coronary vessels using computational fluid dynamics. The computational fluid dynamics implements said at least one patient-specific boundary condition and said at least one physical parameter for describing blood as a flow medium. Said implementation allows to obtain patient-specific results that are useful in diagnosis.

In preferred embodiments of the invention, the fluid domain ($\Omega_f$) may satisfy the following equation

$$\begin{cases} \rho\left(\dfrac{\partial \mathbf{u}}{\partial t} + \mathbf{u} \otimes \nabla \mathbf{u}\right) - \eta\Delta\mathbf{u} + \nabla p = 0, \\ \qquad\qquad \nabla\mathbf{u} = 0 \end{cases}$$

for which: $\rho$ -fluid density, $t$ -time coordinate, $\mathbf{u}$ and $p$ flow velocity and pressure, $\eta$ is dynamic viscosity coefficient.

In preferred embodiments of the invention, the porous domain ($\Omega_p$) may satisfy the following equation

$$\nabla p = -\frac{\eta}{\mathbf{K}}\mathbf{u} - \frac{\rho C_F}{\sqrt{\mathbf{K}}}|\mathbf{u}|\mathbf{u},$$

for which: $\rho$ -fluid density, $\mathbf{u}$ and $p$ -flow velocity and pressure, $\eta$ -dynamic viscosity coefficient, $C_F$ - inertial resistance tensor, K -permeability tensor.

In preferred embodiments of the invention, the flow through the fluid domain ($\Omega_f$) and the porous domain ($\Omega_p$) may satisfy the following equation:

$$\rho\left(\frac{\partial \mathbf{u}}{\partial t} + \mathbf{u} \otimes \nabla\mathbf{u}\right) - \eta\Delta\mathbf{u} + \nabla p + \chi\left(\frac{\eta}{\mathbf{K}}\mathbf{u} + \frac{\rho C_F}{\sqrt{\mathbf{K}}}|\mathbf{u}|\mathbf{u}\right) = 0, in\ \Omega_f \cap \Omega_p,$$

for which $\chi$ = 1 for the porous domain ($\Omega_p$), or 0 elsewhere,

For certain embodiments, in the porous domain, the Navier-Stokes equation are supplemented with additional terms associated with the resistance induced by a porous medium. The additional terms are based on the Forchheimer model describing both the viscous and inertial drag effects. This combination of the Navier-Stokes equation with the Forchheimer model allows to by-pass the extreme geometrical complexity of the porous domain, while it is able to recreate the nature of the phenomena taking place in said domain. In this inventive approach, information of key importance for diagnostic purposes, i.e., information about a flow distribution, is directly extracted. In other words, this unique feature allows to arrive at a relevant information without the time and effort needed to build and to solve a mathematical model of a porous domain. This is beneficial in terms of a need for computational power and in terms of pace of calculations.

The present invention involves calculating of perfusion metrics which can include in embodiments:

1) myocardial blood flow (MBF) which can be defined as the volume of blood transiting through a myocardium tissue at a certain rate,

2) myocardial blood volume (MBV) which can be defined as the total volume of blood in a given unit volume of a myocardium,

3) mean transit time (MTT) which can be defined as the expected value of time interval that blood spends within a particular part of a myocardium, and

4) time to peak (TTP) which can be defined as the interval of time in radiodensity or as the interval of time in which an indicator of the concentration will be at its maximum in a particular part of a myocardium.

The invention allows for calculating of MBF, MBV, MTT and TTP directly or in the form of indexes, ratios, regional values or patient-specific reference values.

Said perfusion metrics can be calculated using regional tracer kinetic recovery methods or using sequential Monte Carlo streamline trajectory Lagrangian tracking. In particular, calculation of the perfusion metrics can involve MaXimum Slope (MXS), Deconvolution of Impulse Response Function (DRF), multi-pathway, multi-species and non-linear Blood-Tissue eXchange (BTX). In other embodiment, calculation of the perfusion metrics can involve

$$r_n = r_1 + \sum_{i=1}^{n-1} \Delta s_i$$

wherein $s_i$ are train vectors $\{\Delta s_1, \Delta s_2, \ldots, \Delta s_n\}$, where $\Delta s_i = f(t, r_i)$.

Further characteristics and advantages of the invention will be more apparent from the detailed description of nonlimiting embodiments and from the appended figures. It should be understood that embodiments presented in the description and in the claims, unless stated otherwise, can be combined together in any order and number to produce new embodiments that form part of the disclosure.

BRIEF DESCRIPTION OF DRAWINGS

The invention will now be described in more detail with reference to the appended figures in which:

FIG. 1 is presenting a processing flow chart incorporating steps of a method according to an embodiment of the present invention,

FIG. 2 is presenting a myocardial perfusion which was computed using actual tomography results. FIG. 2 (A) is presenting tissue attenuation and arterial input curves and FIG. 2 (B) is presenting a long and a short axis view of MBF and TTP,

FIG. 3 is a three-dimensional reconstruction of a heart muscle and coronary arteries from computed tomography angiographic projections. Each of the marked points on a reconstructed three-dimensional mesh in an angiographic projection represents an identified stenosis and their counterparts,

FIG. 4 is presenting an unstructured tetrahedral three-dimensional mesh of a heart muscle and coronary arteries,

FIG. 5 is a visualization of a normalized flow rate field (A, B) and of a pressure field (C, D) computed with a numerical simulation of blood flow, and

FIG. 6 is a 17-segment AHA (American Heart Association) model visualization of numerical simulation results of a myocardial perfusion (B) compared with a reference myocardial blood flow results computed from a CTP (A) for the same human patient. Locations of coronary arteries were marked on both images.

DETAILED DESCRIPTION

While this description often refers to engineering as a source of medical approach used in the invention, there are significant differences between the engineering approach and the medical approach. Said differences are visible for a person skilled in the art and are not limited to different naming used in both fields. For example, certain engineering measurements can be made directly since, in a given example, we can measure a flow in a pipe or in a river and the shape of the pipe or the river does not change significantly during the measurement. On the other hand, a heart is undergoing cyclic significant changes but there is no indication in which part of a cycle a given measurement is made. This limited example is only to sketch the differences between engineering and medicine. This description of the invention is not meant to explain all aspects of engineering and medical convention used herein as this should be understandable for the skilled person.

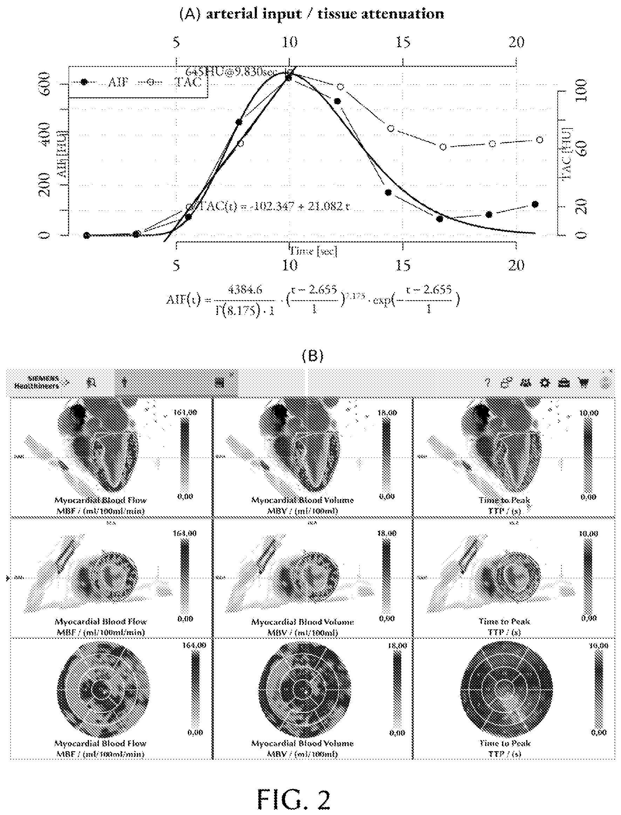

The indicator-dilution methods used in cardiovascular diagnostics, as well as the well-known engineering dye-dilution, tracer-dilution or even the Allen's salt-velocity method, derive from one very elementary concept binding the three conventional scales: time, volume and mass. Labelling them - conventionally - as T, V, M we have T=V/Q (Q - volumetric flow rate), M/V=C (C - concentration of indicator) while in view of the change of M as a function of time we have the relation dM/dt=Q·ΔC (following Peters AM (1993) A unified approach to quantification by kinetic analysis in nuclear medicine. J Nucl Med. 34(4):706-13). The whole analysis concerning these three conventional scales and determined diagnostic parameters such as MBF, MBV, MTT, TPP by the software accompanying CT scanners is implemented according to equations (1)...(25). The result of an *in vivo* imagining comprises a series of images, usually saved in a DICOM format. The results and its particular format allow for reconstruction of TAC and AIF curves for a number of time points. Each time point is associated with dozens of images that must be saved by the device in order to reconstruct the tissue time attenuation curve. This can be technically demanding to be done within a split of a second or even within the time needed to record a single heart cycle. In fact, the time points on the timeline are quite remote from each other and only their mutual time interval is measured by tracking the patient's ECG (electrocardiogram). A representative example of this is FIG. 2 which shows the result of medical examination of a patient: in its upper part (A) one can see AIF (filled circles) and TAC (empty circles) curves and their approximations for the whole myocardium (here: volume of interest, VOI), while in its lower part (B) the distribution of MBF and TTP in the cardiac axis planes. Using the maximum slope method (see equation 22 above), which was implemented for the results shown in FIG. 2, it is necessary to know max(AIF) and max(dTAC/dt) to determine MBF. This task is challenging and the first value is determined by approximating an empirical AIF curve (the equation under the horizontal axis) due to numerous perturbations. In comparison, it is even more challenging to find max(dTAC/dt). This is usually achieved by determining the angular slope of the tangent, as shown by the equation in the graph TAC(t) =-102.347+21.082·t (the tangent itself is drawn with a bold line). What can be clearly seen is that the tangent is built using only three points. It is quite rare to use more than four points for this purpose. As is may be seen in AIF and even more so in TAC curves, *in vivo* radiodensity curve varies locally from the expected curve. Local variations are not the only one as, in fact, the whole curve may deviate from the expected curve. As can be seen, the radio-density values unexpectedly do not decrease but, after reaching a local minimum, gradually increase. While this kind of effect would be difficult to observe on a global level, locally for a single voxel it is a common phenomenon and has its origin in recirculation of an indicator (contrast) substance.

The idea of estimating the degree of circulatory insufficiency on the basis of an MPI examination is laudable and over time will certainly become a new gold standard for diagnostic tests. However, its implementation which is based on what was developed in engineering (e.g., in civil engineering or groundwater hydraulics) does not seem to lead to success. This can be explained. A heart is an organ that is subject to a constant motion and which is experiencing cyclic contractions. It is not possible, even for a few minutes, to stop a human heart for the needs of a diagnostic procedure. On the other hand, technical limitations of the diagnostic procedure do not allow to increase a time resolution significantly. Already at a very early stage of application of perfusion diagnostic methods, this problem was recognized. For example, a team from the University of Münster concluded: *"Sampling intervals of > 1 second on PCT imaging calculated using software relying on the maximum slope model significantly alter absolute CBF and TTP value..."* (see Kloska SP et al (2010) Increasing sampling interval in cerebral perfusion CT: limitation for the maximum slope model, Acad Radiol, 17(1), p. 61-66). Indeed, due to the interrelationship of time scales of a cardiac cycle and acquisition, and furthermore due to the magnitude of a volume of distribution, the derivative of dTAC/dt must be calculated from a section of the TAC curve which in turn is built using two, three, or four time point-layers. Even worse, the injection site for a contrast agent is usually far away from the target organ and the duration of the injection must be sufficiently long relative to the other time scales (which is required for the well-being of a patient).

In an embodiment of the present invention, it is permitted to use the maximum slope method (MXS) and, particularly, the relations (22)-(25) for recovery of regional tracer kinetics (TKR).

In another and preferred embodiment of the present invention, the recovery of regional tracer kinetics (TKR) will be based on an assessment of how the tracer is retained in the vasculature which is done instead of tracer rates. If, within a time interval $\Delta t$, a $\Delta m(t)$ of a contrast agent from the total amount m introduced at the inlet has flows into the outlet region of interest (ROI), the empirical probability of such an event is expressed by

$$P(t \in \Delta t) = \frac{\Delta m(t)}{m} \ . \tag{26}$$

In this way, we can construct an empirical probability density function $h(t)$ which is relating the probability value $P(t \in \Delta t)$ with the width of the interval $\Delta t$

$$h(t) = \frac{P(t)}{\Delta t}, t \in \Delta t \ . \tag{27}$$

Knowing the distribution of the probability density function $h(t)$ over the time over which fractions of the tracer substance reach the outlet, the empirical cumulative distribution function can be easily determined using the following equation

$$H(t) = \int_0^t h(\tau)d\tau \ , \tag{28}$$

and the probability of the opposite event consisting of an event where time exceeds the assumed value of $t$

$$R(t) = 1 - H(t) \ . \tag{29}$$

Mathematical description can be presented as: If a tracer in an amount m is introduced at the inlet in an infinitely short time interval, then at any time t an amount $H(t) \cdot m$ reaches the ROI volume, and there is still $R(t) \cdot m$ of the tracer remaining outside of the ROI. In the real-world conditions, the tracer is introduced in fixed portions, over a finite time interval $\Delta t_i$, often at a distal location, and it slowly flows with the blood to the presumed ROI. Then, the concentration change recorded by the TAC(t) curve induced by an earlier injection at the inlet at time $\Delta t_i$ (which corresponds to the inlet value AIF($t_i$)), will be expressed by: $TAC(t) = AIF(t) \cdot T(t - t_i) \cdot MBF \cdot \rho_t \cdot \Delta t_i$. If we treat the gradual changes in the concentration recorded at the arterial input function as a series of injections at times $t_1, t_2, \dots, t_n$ with durations $\Delta t_1, \Delta t_2, \dots, \Delta t_n$, then at time t the concentration in the ROI volume will be expressed by

$$TAC(t) = \sum_{i=1}^n AIF(t_i) \cdot R(t - t_i) \cdot MBF \cdot \rho_t \cdot \Delta t_i \ , \tag{30a}$$

and consequently, with sufficiently large $n \to \infty$, the concentration in the target region of interest will change to

$$TAC(t) = \int_0^t AIF(\tau) \cdot \rho_t MBF \ R(t - t) \cdot dt \ . \tag{30b}$$

By introducing the impulse response function $IRF(t) = \rho_t \cdot MBF \cdot R(t)$, it is easy to see that the resulting product integral defines a convolution operation which can be expressed by

$$TAC(t) = \int_0^t AIF(\tau) \cdot IRF(t - \tau) \cdot d\tau = AIF(t) \otimes IRF(t) \ . \tag{31}$$

The resulting equation is the central element of the secondary and preferred embodiment of the present invention. This equation defines a concise transformation principle of the state observed at the input to the state in the arbitrary region of interest and this may be expressed by:

$$AIF(t) \xrightarrow{IRF} TAC(t) \ . \tag{32}$$

Awareness of impulse response function $IRF(t)$ greatly facilitates the assessment of perfusion parameters (perfusion metrics). Note that since max($R(t)$) = 1, then according to the definition max($IRF(t)$) = $\rho_t \cdot MBF$, hence the myocardial blood flow $MBF = $ max(IRF $(t)$) / $\rho_t$. By summing the equation (31) over a long period of time, by virtue of Fubini's theorem we arrive at the following equation

$$\int_0^\infty TAC(t)dt = \int_0^\infty AIF(t) \otimes IRF(t)dt = \rho_t MBF \cdot \int_0^\infty AIF(t)dt \cdot MTT , \qquad (33)$$

since $MTT = \int_0^\infty t \cdot h(t)dt = \int_0^\infty (1 - H(t))dt = \int_0^\infty R(t)dt$, but *MBF = MBV/MTT*. The above-presented approach is part of DRF.

In a third preferred embodiment of the present invention, recovery of regional tracer kinetics (TKR) is carried out through a blood-tissue exchange model (BTX) (following Wirestam R (2012) Using contrast agents to obtain maps of regional perfusion and capillary wall permeability, Imaging in Medicine, 4(4), p. 423-438, Bindschadler M et al. (2014) Comparison of blood flow models and acquisitions for quantitative myocardial perfusion estimation from dynamic CT, Phys Med Biol, 59(7), p. 1533-56, Tofts PS et al. (1999) Estimating kinetic parameters from dynamic contrast-enhanced T(1)-weighted MRI of a diffusable tracer: standardized quantities and symbols, J Magn Reson Imaging, 10(3), p. 223-32). The previously presented TKR models, in the context of pharmacokinetic and pharmacodynamic (PK/PD) modeling, are one-compartment models. Indeed, it would be easy to build a corresponding model of blood-tissue exchange processes on top of a n-compartment PK/PD model. For example, a two-compartment exchange model may be defined as follow

$$\begin{cases} v_e \dfrac{dc_p}{dt} = \rho MBF(1 - HCT)c_{ap} + \rho PS\, c_e - \rho(MBF(1 - HCT) + PS)c_p \\ \qquad v_e \dfrac{dc_e}{dt} = \rho PS \left(c_p - c_e\right) \end{cases} , \qquad (34)$$

where *c* is tracer concentration (*p*-plasma, *e*-extracellular, extravascular space, *ap*-arterial plasma) *v* is fractional plasma volume in a compartment, *PS* is permeability-surface area product. When the effect of the tracer in the extravascular space is negligible, in an embodiment $c_t = v_e c_e$ and $dc_t/dt = v_e dc_e/dt$.

The present invention comprises *in silico* determination of perfusion parameters (perfusion metrics) MBF, MBV, MTT and TTP with the use of computational fluid dynamics (CFD) methodology. In a particular embodiment of the present invention, said determination is realized with the use of one of the regional tracer kinetic recovery (TKR) methods: MaXimum Slope (MXS), Deconvolution of Impulse Response Function (DRF), multi-pathway, multi-species, non-linear Blood-Tissue eXchange (BTX). The use of any of the listed methods is connected with an assumption of familiarity in time evolution of a marker concentration in the volume ROI in every point (or voxel), providing metrics TAC(t) being the result for arterial input. In the case of *in vivo* testing, time evolution of a marker concentration in the volume ROI in every point (or voxel) is provided straightforwardly by imaging results and presented in Hounsfield units (CFD does not present the same and CFD provides pressure and velocity distribution). After images co-registration and motion artifacts removing, a tissue attenuation curve in the volume of interest (VOI) voxel per voxel is reconstructed. This commonly adopted strategy is used by every producent of a perfusion diagnostic device because it is the easiest. The easiest strategy cannot cover all relevant cases and conditions. As it was mentioned earlier, factors that affect measurements are causing, especially in this case of this commonly adopted strategy, differences between the obtained results with different devices and / or with different protocols and / or with different cutoff values for MBF, MBV, MTT or TTP. The variation between the results provided by different researchers may by of dozens of precents (sometimes it is more than 100%). Additionally, the results obtained using this way do not have any direct physical interpretation which may be expressed in terms of e.g., established scales of flow, volume or time. Moreover, the results have really a loose connection with flow, volume or time as such. In consequence, more and more researchers are interested in the subject of *in vivo* perfusion imaging via advection-diffusion (see Liu P et al. (2021) Perfusion imaging: An advection diffusion Approach, IEEE Transactions on Medical Imaging, 40(12), p. 3424-3435). If with the flow rate any substance is transported, then its local concentration change $\partial c(t, r)/\partial t$ is described by advection-diffusion transport equation (Bird RB et al (2006) Transport phenomena, John Wiley & Sons) which may be expressed as

$$\frac{\partial c}{\partial t} + \nabla(\mathbf{j}) = q , \qquad (35a)$$

where q is source term, while flux (to be precise, a flux density as a rate of quantity per unit area) is expressed as

$$\mathbf{j} = \mathbf{u} \cdot c - D \cdot \nabla c . \qquad (35b)$$

In a preferred embodiment of the present invention, marker concentration field distributions can be determined based on the above-mentioned advection-diffusion transport equation. To be more precise, by solving the equation numerically. In the case of an *in vivo* imaging results analysis, which is opposite to an *in silico* method being subject of the present invention, a velocity field does not have to be calculated but can be relatively easily reconstructed based on the imaging results. It is hard to estimate how this approach is going to be recognized by the producents of perfusion *in vivo* imaging devices. In any case, this approach can be very inspiring for development of *in silico* methods. In this case, a velocity field cannot be reconstructed but has to be calculated using a separate model. From the standpoint of engineering, the description of a marker flow in a tissue can be understood as a description of a marker flow in a porous medium or saturated porous medium. Usually, the description of this flow can implement a Henry Darcy equation (see Ingham D, Pop I (2005) Transport phenomena in porous media, Vafai K (2015) Handbook of porous media, CRC Press) which has a form of

$$\mathbf{u} = -\frac{\mathbf{K}}{\eta} \nabla p \,, \tag{36}$$

where $\mathbf{K}$ is a permeability tensor. Considering that a velocity field satisfies a Euler balance equation (for liquids), a differential equation can be obtained in the form of

$$\nabla \mathbf{u} = \nabla \left( -\frac{\mathbf{K}}{\eta} \nabla p \right) = 0 \,. \tag{37}$$

This equation gives the possibility of calculation a pressure distribution in a porous domain. Once we arrive at pressure (p), we can use the Darcy equation to obtain the velocity $\mathbf{u}$ (see Alves JR et al (2019) Simulation of the perfusion of contrast agent used in cardiac magnetic resonance: a step toward non-invasive cardiac perfusion quantification, Front Physiol, 10, p. 177, Alves JR et al. (2016) Simulation of cardiac perfusion by contrast in the myocardium using a formulation of flow in porous media, J. Comput. Appl. Math, 295(C), p. 13-24). Every differential equation case, including equation (36), requires boundary condition (here: pressure) to be solved. In every case - also in a preferred embodiment of the present invention - a pressure distribution on the edge of a porous heart muscle is affected by the inflow from coronary arteries. This observation can be generalized for perfusion models of different organs. In a simplified approach, one might use a Daniel Bernoulli equation to achieve this goal. (following Vladimir Lukes et al. (2014) Numerical simulation of liver perfusion: from CT scans to FE model, CoRR, abs/1412.6412). A more advanced approach to the modeling of an inflow to myocardium is presented in the work of a French-American team (see Papamanolis L et al. (2021) Myocardial perfusion simulation for coronary artery disease: a coupled patient-specific multiscale model, Ann Biomed Eng, 49(5), p. 1432-1447, Jaquet C (2018) Toward myocardium perfusion from x-ray computed tomography, Ph.D. Thesis, Université Paris Est) which developed the following equations

$$\begin{cases} \dfrac{\partial Q}{\partial z} = 0 \\ \dfrac{\partial}{\partial z}\left( \alpha \dfrac{Q^2}{S} \right) + \dfrac{S}{\rho}\dfrac{\partial p}{\partial z} + 8\pi\nu\dfrac{Q}{S} = 0 \end{cases} . \tag{38}$$

Said equations are describing the coronary flow as a steady blood flow along the centerline axis of each vessel. In fact, models implementing said equations are well known to any engineer studying available textbooks about hydraulics (see Wylie EB, Streeter VL (1967) Hydraulics Transients. McGraw-Hill). It is worth to note that this approach to the description of a coronary flow can be easily and effectively expanded by introducing inertial effects. A good example is the work of a Siemens Corporate Research and Technology team (see Sharma P et al. (2012) A patient-specific reduced-order model for coronary circulation, 2012 9th IEEE International Symposium on Biomedical Imaging, p. 832-835, Formaggia L et al. (2003) One-dimensional models for blood flow in arteries, Journal of Engineering Mathematics, 47, p. 251-276). Nevertheless, in its original form, so far, it has not been used in the context considered here. Equation 38 is not used in the invention.

In a preferred embodiment of the present invention, evolution of velocity ($\mathbf{u}$) and pressure (p) fields is described by a system of Euler mass balance and Navier-Stokes momentum equations. A myocardium (and in general a muscle tissue) supported by a network of branched arteries can be considered to be two non-overlapping and simply connected domains $\overline{\Omega} = \overline{\Omega}_f \cup \overline{\Omega}_p$, $\Omega_f \cap \Omega_p = \emptyset$ where $\overline{\Omega}_f$ is the closure of fluid domain $\Omega_f$ and represents the fluid domain $\Omega_f$ with boundary $\Gamma_f$, while $\overline{\Omega}_p$ represents a saturated porous media domain $\Omega_p$ with boundary $\Gamma_p$. An interface between the two domains can be indicated by $\Gamma = \overline{\Omega}_f \cap \overline{\Omega}_p \neq \emptyset$. In the fluid region $\Omega_f$, the blood movement is treated as the transient flow of an incompressible medium described by Euler mass balance and Navier-Stokes momentum equations (following Bird RB et al (2006) Transport phenomena, John Wiley & Sons, Pozrikidis C (2011) Introduction to theoretical and computational fluid dynamics, Oxford University Press, Chung TJ (2010) Computational Fluid Dynamics Cambridge University Press) of the form

$$\begin{cases} \rho\left(\dfrac{\partial \mathbf{u}}{\partial t} + \mathbf{u} \otimes \nabla\mathbf{u}\right) - \eta\Delta\mathbf{u} + \nabla p = 0 \quad in\ \Omega_f \\ \nabla\mathbf{u} = 0 \end{cases} \tag{39}$$

It is a common general knowledge that blood forms a polydisperse system comprising a liquid phase and a suspended dispersed phase. The liquid phase is plasma and the suspended dispersed phase is created from morphotic components: erythrocytes (which are the most numerous and responsible for a gas exchange), leukocytes (whose numbers are low and which provide immunity functions) and thrombocytes (which are actively involved in coagulation processes, ensuring haemostasis). In average conditions, a blood density is in the range of $p_f = 1.05\ ...1.06$ g/cm$^3$. A blood density is generally decreasing with age (reaching its maximum for newborns). A blood density can be more precisely determined as a function of a volume fraction of morphotic elements present in human blood. For example, the Hawksley formula is often used to estimate blood density: $\rho_f = \rho_{pl}(1 - HCT) + HCT\rho_{RBC}$, where $\rho_{pl}$, $\rho_{RBC}$ is density of plasma and erythrocytes, and HCT is a hematocrit (see De Gruttola S et al. (2005) Computational simulation of a non-newtonian model of the blood separation process, Artif Organs, 29(12), p. 949-59). Estimation of a blood viscosity coefficient is much more complex task in comparison to a blood density (following Milnor WR (1982) Hemodynamics, Williams&Wilkins, Vlachopoulos Ch et al. (2011) McDonald's blood flow in arteries, CRC Press, Kannojiya V et al. (2021) Simulation of Blood as Fluid: A Review from Rheological Aspects, IEEE Rev Biomed Eng, 14, p. 327-341). First of all, the coefficient depends on the content of the solid fraction, which is considered to be typical for dispersion systems. For blood, the coefficient is expressed as the value of the hematocrit number (HCT). Moreover, red blood cells have a unique ability to create three-dimensional structures in the form of rouleaux (see Samsel RW, Perelson AS (1982) Kinetics of rouleau formation. I. A mass action approach with geometric features, Biophys J, 37(2), p. 493-514, Samsel RW, Perelson AS (1984) Kinetics of rouleau formation. II. Reversible reactions, Biophys J, 45(4), p. 805-2). In a preferred embodiment of the present invention, blood rheological properties to be used in computational fluid dynamics are described using a model which is of the Williamson class (Williamson fluid model), for which $(\eta - \eta_\infty)/(\eta_0 - \eta_\infty) = F(HCT,\dot{\gamma})$. The structural function of the $F$ model in a preferred embodiment is selected according to the Carreau, Cross, Yasuda model (see Carreau PJ (1972) Rheological equations from molecular network theories, Journal of Rheology, 16(1), p. 99-127, Cross MM (1965) Rheology of non-Newtonian fluids: A new flow equation for pseudoplastic system, Journal of Colloid Science, 20(5), p. 417-437, Yasuda K (1979) Investigation of the analogies between viscometric and linear viscoelastic properties of polystyrene fluids, Ph.D. Thesis, Massachusetts Institute of Technology). In another embodiment, it is also possible to use the Walburn-Schneck model, which explicitly takes into the account the influence of blood plasma proteins (see Walburn FJ, Schneck D (1976) A constitutive equation for whole human blood, Biorheology, 1, p. 201-210).

While it is customary for engineers to express the movement in porous domain $\overline{\Omega}_p$ by the Darcy equation (see eqn. (36)), this equation has been specifically rejected and it is not implemented in the present invention. This equation does not include inertial effects in its structure (in principle. it can only be used within the Reynolds $Re_p < 1$). Instead, a non-linear equation is used in a preferred embodiment of the present invention (following Forchheimer P (1901) Wasserbewegung durch Boden, Z. Ver. Deutsch. Ing, 45(1901), p. 1782-1788, Whitaker S (1996) The Forchheimer equation: A theoretical development, Transport in Porous Media, 25, p. 2761, Vafai K (2015) Handbook of porous media, CRC Press) which has the form of

$$\nabla p = -\frac{\eta}{\mathbf{K}}\mathbf{u} - \frac{\rho\, C_F}{\sqrt{\mathbf{K}}}\,|\mathbf{u}|\mathbf{u} \quad in\ \Omega_p, \tag{40}$$

where $C_F$ is an inertial resistance tensor. In order to obtain a consistent phenomenological description, in a preferred embodiment of the present invention penalization approach has been introduced to model the flow through parts of the fluid $\overline{\Omega}_f$ and porous domain $\Omega_p$ (following Bruneau CH, Mortazavi I (2008) Numerical modelling and passive flow control using porous media. Computers & Fluids. 37(5):488-498) with the following equation

$$\rho\left(\frac{\partial \mathbf{u}}{\partial t} + \mathbf{u} \otimes \nabla\mathbf{u}\right) - \eta\Delta\mathbf{u} + \nabla p + \chi\left(\frac{\eta}{\mathbf{K}}\mathbf{u} + \frac{\rho\, C_F}{\sqrt{\mathbf{K}}}\,|\mathbf{u}|\mathbf{u}\right) = 0 \quad in\ \Omega_f \cap \Omega_p, \tag{41}$$

while $\chi = 1$ in a porous domain, or 0 elsewhere.

Since the solution of the above equation provides the full description about the evolution of the pressure fields and about the velocity (in the nodes of mesh used for calculations) and thus about the local values of flow rates in each mesh cell, in another embodiment of the present invention, a myocardial blood flow (MBF) can be calculated directly.

In another embodiment of the present invention, the reconstruction of the parameters describing perfusion can be realized with the use of sequential Monte Carlo streamline trajectory Lagrangian tracking in the form of

$$r_n = r_1 + \sum_{i=1}^{n-1} \Delta s_i \qquad (42)$$

by train vectors $\{\Delta s_1, \Delta s_2, ..., \Delta s_n\}$, where $\Delta s_i = f(t, r_i)$ (see Crane M, Blunt M (1999) Streamline-based simulation of solute transport, Water Resour. Res, 35, p. 3061-3078, Salamon P et al. (2006) A review and numerical assessment of the random walk particle tracking method, J. Contam. Hydrol, 87, p. 277-305, Friman O et al. (2010) Probabilistic 4D blood flow mapping, International Conference on Medical Image Computing and Computer-Assisted Intervention, 13(Pt 3), p. 416-23, Bear J (2018) Modeling phenomena of flow and transport in porous media, Springer International). This form of post-processing of the calculation results enables to determine distributions of the probability density function $h(t)$, the distribution function $H(t)$ and the residual function $R(t)$, in accordance with the equations (27) - (29). In this way, it is possible to use any of the above-mentioned methods for calculating perfusion parameters based on the results of CFD simulations.

An embodiment of the method of the present invention comprises six steps: 1) collection of patient-specific demographic and general medical data and patient-specific anatomical structure data, 2) quantification of boundary condition, 3) creation of a discrete model of geometry, 4) numerical simulation in fluid and porous domains, 5) recovery of regional perfusion metrics, and - finally - 6) visualization of the regional perfusion metrics.

In the first step, patient-specific demographic and general medical data are collected. In particular, patient-specific demographic and general medical data include age, sex, height, weight, systemic blood pressure and / or blood test results (if available, hematocrit number in particular) of a particular patient. The patient's demographic and general medical data may further include information on a patient's pharmacological therapy. The patient's pharmacological therapy may include a beta-adrenergic blocking agent, an angiotensin-converting-enzyme inhibitor and/or an antiarrhythmic agent. Other embodiments comprise other pharmacological therapies. It should be noted that pharmacological therapies include also different dosing regimes as well as any further medical effects of drug therapies. It is contemplated that in certain embodiments only selected patient-specific demographic and general medical data are collected, e.g., gender, age or pharmacological therapies. The patient-specific demographic and general medical data can be collected via an interview with a patient or can be obtained from a database.

In addition to patient's demographic and general medical data, which are subsequently given a physical interpretation, patient-specific anatomical structure data are also collected. In one embodiment, the patient-specific anatomical structure data comprise the results of a computed tomography angiography (CTA) of the coronary arteries and myocardium. In other embodiments, computed tomography and / or magnetic resonance techniques can be used. The patient-specific anatomical structure data comprise information about anatomy of a heart myocardium and vessels connected to the myocardium. The patient-specific anatomical structure data can be collected before, after or in parallel with the collection of the patient-specific demographic and general medical data. The patient-specific anatomical structure data can be retrieved from a database.

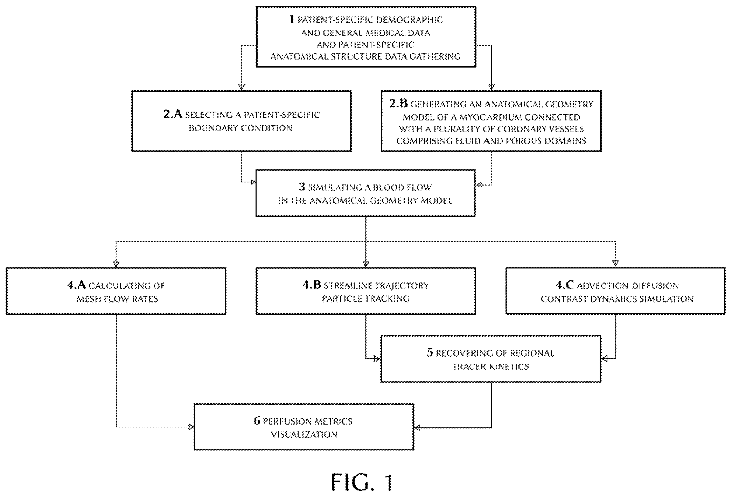

In the second step, the patient's demographic and general medical data is used to determine one or more patient-specific boundary conditions, as well as to select one or more physical parameters that will be describing blood as a flow medium. This is needed for a numerical simulation. The selected physical parameters may include a density and/or a dynamic viscosity coefficient. In the diagram of FIG.1, this is represented by 2.A. In a preferred embodiment, at the same time, the imaging results from the CTA, preferably in the form of a series of several hundred DICOM images, are segmented, which ultimately provides a surface model of the arteries and muscles of a patient's heart. This is preferable as it allows to arrive at the results of the method faster. In other embodiment, which are less computational demanding, segmentation may be done before or after determination of said one or more patient-specific boundary conditions. In the diagram of FIG. 1, this is represented by 2.B.

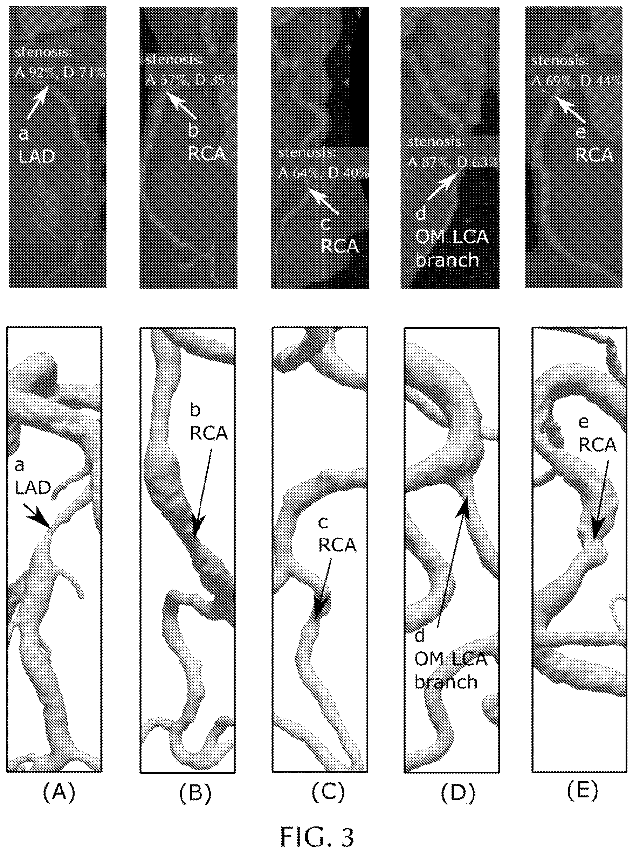

As an example, FIG. 3 shows the direction of the main coronary arteries visualized by a curved planar reconstruction (CPR) of the artery along a centerline (CPR view). A skilled person knows that a curved planar reconstruction is sometimes called a curved planar reformation. The case presented on FIG. 3 had two strictures on the left branch (a: 92% and d: 87%, OM branch) and three on the right branch (b: 57%, c: 64%, e: 69%). In the upper part of the figure, the CPR view can be seen, and in the lower part of the figured there is enlarged the result of the segmentation and reconstruction of the surface model (again, with the location of the strictures marked).

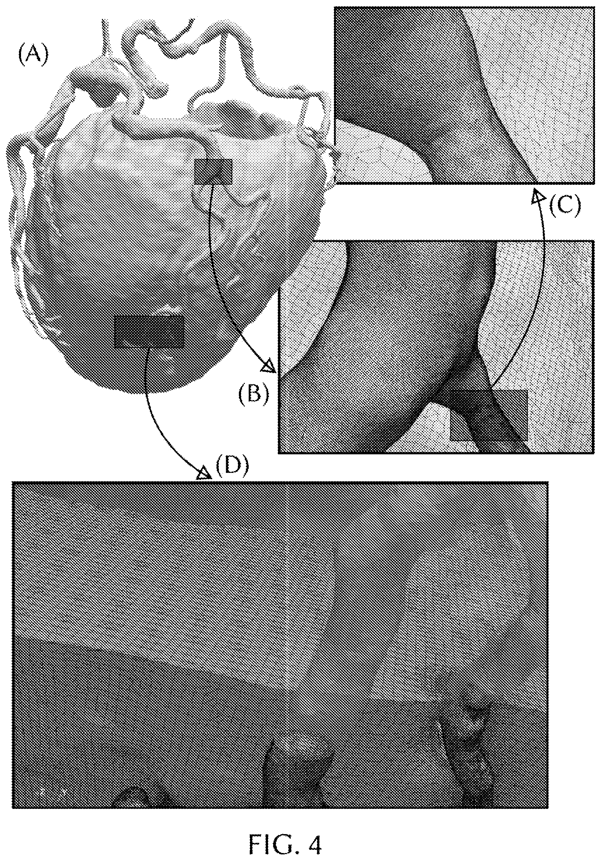

The resulting surface model is discretized through an unstructured volumetric mesh which is shown in FIG. 4. In the upper-left corner of FIG. 4, we have a complete fluid and porous domains computational mesh view, the remaining parts of the figure show the enlarged artery and myocardium mesh. In addition, the changes in radio density recorded in the CTA are used to quantify the perfusion features of the myocardium (following Vinnakota KC, Bassingthwaighte JB (2004) Myocardial density and composition: a basis for calculating intracellular metabolite concentrations, American Journal of Physiology-Heart and Circulatory Physiology, 286(5), p. H1742-H1749, Gheorghe AG et al. (2019) Cardiac left ventricular myocardial tissue density, evaluated by computed tomography and autopsy, BMC Medical Imaging, 19, p. 29, Cuong NLQ et al. (2018) Porosity estimation from high resolution CT SAN images of rock samples by using Housfield unit, Open Journal of Geology, 8, p. 1019-1026, Sudhyadhom A (2020) On the molecular relationship between Hounsfield Unit (HU), mass density, and electron density in computed tomography (CT), PLoS ONE 15(12), p. e024486).

In third step, a computational grid with the conditions set on its edge (boundary conditions) and with the specification of the fluid together with porous domain physical properties are transferred to a solver engine. As presented earlier, two sets of equations are solved, albeit in a penalized approach. The equations of fluid motion in the form of a system of Euler and Navier-Stokes equations are solved for the entire computational grid. A skilled person will understand that the entire computational grid excludes boundary conditions which are put to arrive at a specific solution. In a porous domain, the Navier-Stokes equation are supplemented with additional terms associated with the resistance induced by a porous medium. The additional terms are based on the Forchheimer model describing both the viscous and inertial drag effects. This combination of the Navier-Stokes equation with the Forchheimer model allows to by-pass the extreme geometrical complexity of the porous domain, while it is able to recreate the nature of the phenomena taking place in said domain. In this inventive approach, information of key importance for diagnostic purposes, i.e., information about a flow distribution, is directly extracted. In other words, this unique feature allows to arrive at a relevant information without the time and effort needed to build and to solve a mathematical model of a porous domain. This is beneficial in terms of a need for computational power and in terms of pace of calculations. At the same time, it should be emphasized that building an accurate complex model of a heart myocardium connected with a plurality of coronary vessels without the use of a fluid and porous domain may not always be possible.

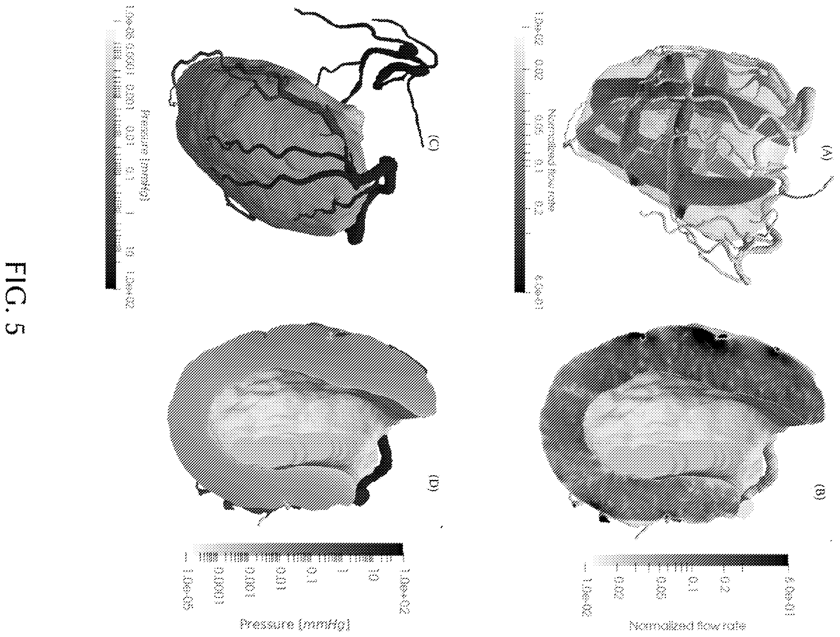

In the fourth step, a diagnostic interpretation by algorithms is given to the results obtained with the use of numerical fluid dynamics methods. Note that, regardless of the type and degree of complexity, the numerical fluid dynamics models only provide information about the velocity and pressure field distributions in the flow. These results are not directly used in medical diagnosis. When assessing perfusion, the overall expectation is that the diagnostic method will provide some contractual metrics such as MBF, MBV, MTT, TTP, or somehow related thereto (such as relative index values). The fourth step can be carried out in three variants, differing in computational inputs and achieved results. In the most direct approach (FIG. 1 - step 4.A), the flow and pressure flow distributions are determined as shown in FIG. 5 - in view and in cross section of the long axis of the heart. However, this method does not provide information on customary perfusion metrics like MBF and the like. Therefore, in the second variant of post-processing of the simulation results, the sequential Monte Carlo streamline trajectory Lagrangian tracking (FIG. 1 - step 4.B) is used. While the usual application of this type of post-processing is the visualization of the trajectory, here it is used to reconstruct the probability distribution functions: density, distribution and residual. This information can be analyzed using known techniques used for analysis of the results of *in vivo* myocardial perfusion imaging. While the effectiveness of particle tracking is high, its accuracy is limited and will not replace full advection-diffusion contrast dynamics simulation. Only this method provides complete information on the flow of contrast in the tissue and is generally equivalent to the *in vivo* methodology. These results (4.C) will undergo the same post-processing as the results of particle tracking (4.B)

In fifth step, reconstruction of the tracer kinetics is performed for the determination of standard perfusion metrics and it is related to the results obtained in step 4.B or 4.C (results from 4.A are not subjected to this kind of processing). It is possible to use any of the tracer kinetic recovery (TKR) strategies discussed earlier in details, i.e., MaXimum Slope (MXS), Deconvolution of Impulse Response Function (DRF), Blood-Tissue eXchange (BTX). The final results are exported for later visualization.

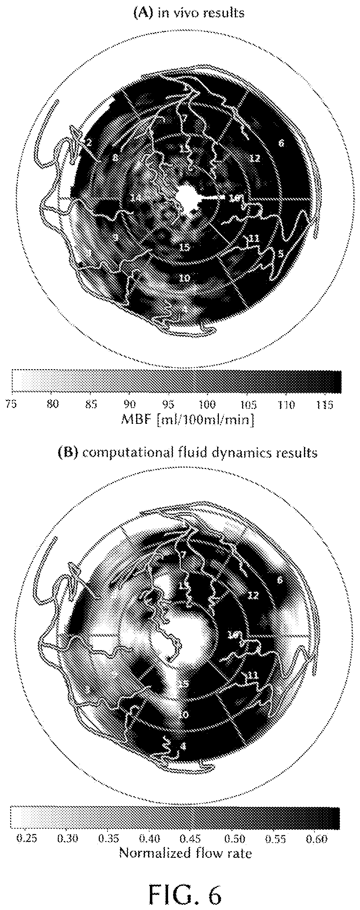

In sixth and last step, only the visualization of previously obtained perfusion metrics is performed. As shown, the aforementioned FIG. 5 visualization may be limited to the presentation of the normalized values of the flows and the pressure distributions. Provided particle tracking or complete contrast dynamics simulation has been performed, the visualization will be for myocardial blood flow (MBF), myocardial blood volume (MBV), mean transit time (MTT), and time to peak (TTP). Such results are shown in FIG. 6 - here - additionally they were placed in the system of the American Heart Association 17-segment model (Cerqueira MD (2002) Standardized myocardial segmentation and nomenclature for tomographic imaging of the heart. A statement for healthcare professionals from the Cardiac Imaging Committee of the Council on Clinical Cardiology of the American Heart Association, Circulation, 105(4), p. 539-42). Because prefusion metrics varies strongly among patients, indexed values may be applied with normalization for interindividual differences (Kono AK et al. (2014) Relative myocardial blood flow by dynamic computed tomographic perfusion imaging predicts hemodynamic significance of coronary stenosis better than absolute blood flow, Invest Radiol, 49(12), p. 801-7, Coenen A et al. (2017) Integrating CT myocardial perfusion and CT-FFR in the work-up of coronary artery disease, JACC Cardiovasc Imaging, 10(7), p. 760-770).

Any calculation step or substep of the method according to the invention is implemented using a computer or a computer program. In some specific embodiments, some or all calculations are done using a computer program stored on a computer or on any type of a memory device or both. In another embodiments, some or all calculations for the purpose of the method can be done remotely e.g., using cloud-based infrastructure which can include the use of Internet or a local network.

While all measurement methods and algorithms described here are intended to define the parameters of the invention and to provide tangible results to be compared with clinical trials, there are by no means limiting and are exemplary. The words like "including" or "comprising" are not limiting and, for example, when an element A includes another element B, the element A may include other element or elements in addition to the element B. The use of a singular or plural form is not limiting for the scope of the disclosure and, for example, a part of the description which is indicating that an element A contains an element B, this part also discloses that multiple elements B are contained in one element A and multiple elements A are contained in one element B as well as an embodiment where multiple elements A contain multiple elements B. Many other embodiments will be apparent and clear for those skilled in the art upon reviewing the content of the present disclosure. The scope of the invention, therefore, should be determined with reference to the appended claims.

**Claims**

1. A computer-implemented method for assessment of a myocardial perfusion using a non-invasive measurement and poromechanics for a human patient, wherein the method comprises:

   - simulating (3) a blood flow in an anatomical geometry model ($\overline{\Omega}$) of a myocardium connected with a plurality of coronary vessels using computational fluid dynamics, wherein the computational fluid dynamics comprises at least one patient-specific boundary condition and at least one physical parameter for describing blood as a flow medium, and
   wherein the anatomical geometry model ($\overline{\Omega}$) of a myocardium connected with a plurality of coronary vessels comprises a fluid domain ($\Omega_f$) and a porous domain ($\Omega_p$) and an interface ($\Gamma$) between the fluid domain ($\Omega_f$) and the porous domain ($\Omega_p$), wherein the anatomical geometry model ($\overline{\Omega}$) of a myocardium satisfies the following equations: $\overline{\Omega} = \overline{\Omega}_f \cup \overline{\Omega}_p$ and $\Omega_f \cap \Omega_p = \emptyset$, and
   wherein $\overline{\Omega}_f$ comprises or consists of the fluid domain ($\Omega_f$) and a boundary ($\Gamma_f$) of the fluid domain ($\Omega_f$) and $\overline{\Omega}_p$ comprises or consists of the porous domain ($\Omega_p$) and a boundary ($\Gamma_p$) of the porous domain ($\Omega_p$), and
   wherein the interface ($\Gamma$) satisfies the following equation: $\Gamma = \overline{\Omega}_f \cap \overline{\Omega}_p \neq \emptyset$, and
   wherein said at least one patient-specific boundary condition is determined using patient-specific demographic and general medical data and said at least one physical parameter for describing blood as a flow medium is selected using patient-specific demographic and general medical data, and
   wherein the anatomical geometry model ($\overline{\Omega}$) of a myocardium connected with a plurality of coronary vessels is generated using patient-specific anatomical structure data of the human patient and wherein the patient-specific anatomical structure data of the human patient are obtained from at least one said non-invasive measurement, wherein the porous domain ($\Omega_p$) is configured to describe flow in a myocardium tissue,
   and wherein the method further comprises:

   - calculating perfusion metrics using the results of the blood flow simulation in the anatomical geometry model ($\overline{\Omega}$) of a myocardium connected with a plurality of coronary vessels.

2. The method of claim 1, wherein the patient-specific anatomical structure data are obtained via at least one of a computed tomography angiography, a nuclear magnetic resonance and/or a computed angiographic.

3. The method of claim 2, wherein the patient-specific anatomical structure data comprise images stored in a DICOM format.

4. The method of any of claims 1-3, wherein generating of the anatomical geometry model ($\overline{\Omega}$) of a myocardium connected with a plurality of coronary vessels comprises segmentation of images comprised in the patient-specific anatomical structure data to arrive at a surface model of the arteries and muscles of a human patient's heart.

5. The method of claim 4, wherein generating (2.B) of the anatomical geometry model ($\overline{\Omega}$) of a myocardium connected with a plurality of coronary vessels further comprises a step in which the surface model is discretized through an unstructured volumetric mesh.

6. The method of any of claims 1-5, wherein the patient-specific demographic and general medical data comprise patient's age, sex, height, weight, systemic blood pressure, blood test results and/or information on the current patient's pharmacological therapy, wherein the current patient's pharmacological therapy is selected from, but not limited to, a beta-adrenergic blocking agent, an angiotensin-converting-enzyme inhibitor and an antiarrhythmic agent.

7. The method of any of claims 1-6, wherein the physical parameter is selected from the group comprising density and a dynamic viscosity coefficient.

8. The method of any of claims 1-7, wherein the porous domain ($\Omega_p$) is saturated.

9. The method of any of claims 1-8, wherein the fluid domain ($\Omega_f$) satisfies the following equation:

$$\begin{cases} \rho\left(\dfrac{\partial \mathbf{u}}{\partial t} + \mathbf{u} \otimes \nabla\mathbf{u}\right) - \eta\Delta\mathbf{u} + \nabla p = 0 \\ \nabla\mathbf{u} = 0 \end{cases}$$

and/or wherein the porous domain ($\Omega_p$) satisfies the following equation:

$$\nabla p = -\frac{\eta}{\mathbf{K}}\mathbf{u} - \frac{\rho C_F}{\sqrt{\mathbf{K}}}|\mathbf{u}|\mathbf{u} \, ,$$

and/or wherein the flow through the fluid domain ($\Omega_f$) and the porous domain ($\Omega_p$) satisfies the following equation:

$$\rho\left(\frac{\partial \mathbf{u}}{\partial t} + \mathbf{u} \otimes \nabla\mathbf{u}\right) - \eta\Delta\mathbf{u} + \nabla p + \chi\left(\frac{\eta}{\mathbf{K}}\mathbf{u} + \frac{\rho C_F}{\sqrt{\mathbf{K}}}|\mathbf{u}|\mathbf{u}\right) = 0 \ \text{ in } \Omega_f \cap \Omega_p \, ,$$

wherein: $\rho$ - fluid density; $t$ - time; $\mathbf{u}$ and $p$ - flow velocity and pressure; $\eta$ - dynamic viscosity coefficient; $\chi = 1$ in $\Omega_p$ or $\chi = 0$ outside $\Omega_p$; $C_F$ - inertial resistance tensor; and $\mathbf{K}$ - permeability tensor.

10. The method of any of claims 1-9, wherein the computational fluid dynamics comprises blood rheological properties described using $(\eta - \eta_\infty)/(\eta_0 - \eta_\infty) = F(HCT,\dot\gamma)$, where $\eta$ is dynamic viscosity coefficient and HCT is hematocrit number.

11. The method of claim 10, wherein the $F(HCT,\dot\gamma)$ is selected according to the Carreau, Cross, Yasuda model or the Walburn-Schneck model.

12. The method of claim 1, wherein said calculating perfusion metrics comprises the use of at least one of regional tracer kinetic recovery methods, wherein the regional tracer kinetic recovery methods comprise MaXimum Slope (MXS), Deconvolution of Impulse Response Function (DRF), multi-pathway, multi-species and non-linear Blood-Tissue eXchange (BTX), and / or wherein calculating perfusion metrics comprises the use of sequential Monte Carlo streamline trajectory Lagrangian tracking in the form of

$$r_n = r_1 + \sum_{i=1}^{n-1} \Delta s_i$$

wherein $s_i$ are train vectors $\{\Delta s_1, \Delta s_2, ..., \Delta s_n\}$, where $\Delta s_i = f(t, r_i)$.

13. The method of claim 12, wherein the regional tracer kinetic recovery methods comprise the following equation:

$$TAC(t) = \int_0^t AIF(\tau) \cdot IRF(t - \tau) \cdot d\tau = AIF(t) \otimes IRF(t) \, ,$$

and/or

$$\begin{cases} v_e \dfrac{dc_p}{dt} = \rho MBF(1 - HCT)c_{ap} + \rho\, PS\, c_e - \rho(MBF(1 - HCT) + PS)c_p \\ v_e \dfrac{dc_e}{dt} = \rho\, PS\, (c_p - c_e) \end{cases} ,$$

and/or

$$\int_0^\infty TAC(t)dt = \int_0^\infty AIF(t) \otimes IRF(t)dt = \rho_t MBF \cdot \int_0^\infty AIF(t)dt \cdot MTT \, ,$$

wherein: $\tau$ - instantaneous time moment at the observational interval [0,t]; *AIF* - arterial input function; *IRF* - impulse response function; *c* - tracer concentration (*p*-plasma, *e*-extracellular, extravascular space, *ap*-arterial plasma); *v* - fractional plasma volume in a compartment; *PS* - permeability-surface area product; t - time; *TAC* - time attenuation curve; *AIF* - arterial input function; *IRF* - impulse response function; *MBF* - myocardial blood flow; and *MTT* - mean transit time.

**14.** The method of any of claims 1-13, wherein the perfusion metrics include:

• myocardial blood flow (MBF) defined as the volume of blood transiting through myocardium tissue at a certain rate,
• myocardial blood volume (MBV) is the total volume of blood in a given unit volume of a myocardium,
• mean transit time (MTT) is the expected value of time interval that blood spends within a particular part of a myocardium, and/or
• time to peak (TTP) represents the interval of time in radiodensity or the interval of time in which an indicator of the concentration will be at its maximum in a particular part of a myocardium, and

wherein the MBF, the MBV, the MTT and/or the TTP are calculated as an absolute value or as a relative value.

**15.** A computer-readable medium comprising instructions which, when executed by a computer, cause the computer to carry the steps of a method defined in any of claims 1-14.


**Patentansprüche**

**1.** Ein computerimplementiertes Verfahren zur Beurteilung einer Myokardperfusion unter Verwendung einer nicht-invasiven Messung und Poromechanik für einen menschlichen Patienten, wobei das Verfahren Folgendes umfasst:

- Simulieren (3) eines Blutflusses in einem anatomischen Geometriemodell ($\overline{\Omega}$) eines Myokards, das mit einer Vielzahl von Koronargefäßen verbunden ist, unter Verwendung der rechnerischen Fluiddynamik, wobei die rechnerische Fluiddynamik mindestens eine patientenspezifische Randbedingung und mindestens einen physikalischen Parameter zur Beschreibung von Blut als Strömungsmedium umfasst, und
wobei das anatomische Geometriemodell ($\overline{\Omega}$) eines Myokards, das mit einer Vielzahl von Koronargefäßen verbunden ist, eine Fluiddomäne ($\Omega_f$) und eine poröse Domäne ($\Omega_p$) und eine Grenzfläche ($\Gamma$) zwischen der Fluiddomäne ($\Omega_f$) und der porösen Domäne) umfasst ($\Omega_p$), wobei das anatomische Geometriemodell ($\overline{\Omega}$) eines Myokards die folgenden Gleichungen erfüllt: $\overline{\Omega} = \overline{\Omega}_f \cup \overline{\Omega}_p$ und $\Omega_f \cap \Omega_p = \emptyset$, und
wobei $\overline{\Omega}_f$ die Fluiddomäne ($\Omega_f$) und eine Grenze ($\Gamma_f$) der Fluiddomäne ($\Omega_f$) umfasst oder daraus besteht und $\overline{\Omega}_p$ die poröse Domäne ($\Omega_p$) und eine Grenze ($\Gamma_p$) der porösen Domäne ($\Omega_p$) umfasst oder daraus besteht, und
wobei die Grenzfläche ($\Gamma$) die folgende Gleichung erfüllt: $\Gamma = \overline{\Omega}_f \cap \overline{\Omega}_p \neq \emptyset$, und
wobei die mindestens eine patientenspezifische Randbedingung unter Verwendung patientenspezifischer demografischer und allgemeinmedizinischer Daten bestimmt wird und der mindestens eine physikalische Parameter zur Beschreibung von Blut als Flussmedium unter Verwendung patientenspezifischer demografischer und allgemeinmedizinischer Daten ausgewählt wird, und
wobei das anatomische Geometriemodell ($\overline{\Omega}$) eines mit einer Vielzahl von Koronargefäßen verbundenen Myokards unter Verwendung patientenspezifischer anatomischer Strukturdaten des menschlichen Patienten erzeugt wird und wobei die patientenspezifischen anatomischen Strukturdaten des menschlichen Patienten aus mindestens einer der nicht-invasiven Messungen erhalten werden,
wobei die poröse Domäne ($\Omega_p$ dazu konfiguriert) ist, den Fluss in einem Myokardgewebe zu beschreiben, und wobei das Verfahren ferner umfasst:

- Berechnen von Perfusionsmetriken unter Verwendung der Ergebnisse der Blutflusssimulation im anatomischen Geometriemodell ($\overline{\Omega}$) eines Myokards, das mit einer Vielzahl von Koronargefäßen verbunden ist.

**2.** Das Verfahren nach Anspruch 1, wobei die patientenspezifischen anatomischen Strukturdaten über mindestens eines von einer Computertomographie-Angiographie, einer Kernspinresonanz und/oder einer Computerangiographie erhalten werden.

3. Das Verfahren nach Anspruch 2, wobei die patientenspezifischen anatomischen Strukturdaten Bilder umfassen, die in einem DICOM-Format gespeichert sind.

4. Das Verfahren nach einem der Ansprüche 1-3, wobei das Erzeugen des anatomischen Geometriemodells eines Myokards $(\overline{\Omega})$, das mit einer Vielzahl von Koronargefäßen verbunden ist, das Segmentieren von Bildern umfasst, die in den patientenspezifischen anatomischen Strukturdaten enthalten sind, um zu einem Oberflächenmodell der Arterien und Muskeln des Herzens eines menschlichen Patienten zu gelangen.

5. Das Verfahren nach Anspruch 4, wobei das Erzeugen (2.B) des anatomischen Geometriemodells eines $(\overline{\Omega})$ Myokards, das mit einer Vielzahl von Koronargefäßen verbunden ist, ferner einen Schritt umfasst, in dem das Oberflächenmodell durch ein unstrukturiertes volumetrisches Netz diskretisiert wird.

6. Das Verfahren nach einem der Ansprüche 1-5, wobei die patientenspezifischen demografischen und allgemeinen medizinischen Daten das Alter, das Geschlecht, die Größe, das Gewicht, den systemischen Blutdruck, die Bluttestergebnisse und/oder Informationen über die pharmakologische Therapie des aktuellen Patienten umfassen, wobei die pharmakologische Therapie des aktuellen Patienten aus einem beta-adrenergen Blockierungsmittel, einem Inhibitor des Angiotensinkonversionsenzyms und einem Antiarrhythmikum ausgewählt ist, ohne darauf beschränkt zu sein.

7. Das Verfahren nach einem der Ansprüche 1-6, wobei der physikalische Parameter aus der Gruppe umfassend Dichte und einen dynamischen Viskositätskoeffizienten ausgewählt ist.

8. Das Verfahren nach einem der Ansprüche 1-7, wobei die poröse Domäne gesättigt $(\Omega_p)$ ist.

9. Das Verfahren nach einem der Ansprüche 1-8, wobei die Fluiddomäne die folgende Gleichung $(\Omega_f)$ erfüllt:

$$\begin{cases} \rho\left(\dfrac{\partial \mathbf{u}}{\partial t} + \mathbf{u} \otimes \nabla \mathbf{u}\right) - \eta\Delta\mathbf{u} + \nabla p = 0 \\ \qquad\qquad \nabla\mathbf{u} = 0 \end{cases}$$

und/oder wobei die poröse Domäne (die folgende Gleichung $\Omega_p$) erfüllt:

$$\nabla p = -\frac{\eta}{\mathbf{K}}\mathbf{u} - \frac{\rho C_F}{\sqrt{\mathbf{K}}}|\mathbf{u}|\mathbf{u}\,,$$

und/oder wobei der Fluss durch die Fluiddomäne $(\Omega_f)$ und die poröse Domäne (die folgende Gleichung $\Omega_p$) erfüllt:

$$\rho\left(\frac{\partial \mathbf{u}}{\partial t} + \mathbf{u} \otimes \nabla \mathbf{u}\right) - \eta\Delta\mathbf{u} + \nabla p + \chi\left(\frac{\eta}{\mathbf{K}}\mathbf{u} + \frac{\rho C_F}{\sqrt{\mathbf{K}}}|\mathbf{u}|\mathbf{u}\right) = 0 \ \text{ in } \Omega_f \cap \Omega_p\,,$$

wobei: $p$ - Fluiddichte; $t$ - Zeit; $\mathbf{u}$ und $p$ - Flussgeschwindigkeit und -druck; $\eta$ - dynamischer Viskositätskoeffizient; $\chi$ = 1 in $\Omega_p$ oder $\chi$ = 0 außerhalb $\Omega_p$; $C_F$ - Trägheitswiderstandstensor; und $\mathbf{K}$ - Permeabilitätstensor.

10. Das Verfahren nach einem der Ansprüche 1-9, wobei die rechnerische Fluiddynamik rheologische Blut-Eigenschaften umfasst, die unter Verwendung von beschrieben werden $(\eta - \eta_\infty)/(\eta_0 - \eta_\infty) = F(HCT,\dot\gamma)$, wobei der dynamische Viskositäts-Koeffizient $\eta$ ist und HCT die Hämatokrit-Zahl ist.

11. Das Verfahren nach Anspruch 10, wobei $F(HCT,\dot\gamma)$ das nach dem Carreau-, Cross-, Yasuda-Modell oder dem Walbum-Schneck-Modell ausgewählt wird.

12. Das Verfahren nach Anspruch 1, wobei das Berechnen von Perfusionsmetriken die Verwendung von mindestens einem der regionalen Verfahren zur tracerkinetischen Wiederherstellung umfasst, wobei die regionalen Verfahren zur tracerkinetischen Wiederherstellung MaXimum Slope (MXS), Deconvolution of Impulse Response Function (DRF), Multi-Pathway, Multi-Species und Nonlinear Blood-Tissue eXchange (BTX) umfassen, und/oder wobei das Berechnen von Perfusionsmetriken die Verwendung von sequentieller Monte Carlo Streamline Trajectory Lagrangian Tracking in Form von

$$r_n = r_1 + \sum_{i=1}^{n-1} \Delta s_i$$

umfassen, wobei $s_i$ Zugvektoren $\{\Delta s_1, \Delta s_2, ..., \Delta s_n\}$ sind, wobei $\Delta s_i = f(t, r_i)$ ist.

**13.** Das Verfahren nach Anspruch 12, wobei die regionalen kinetischen Verfahren zurtracerkinetischen Wiederherstellung die folgende Gleichung umfassen:

$$TAC(t) = \int_0^t AIF(\tau) \cdot IRF(t - \tau) \cdot d\tau = AIF(t) \otimes IRF(t) \ ,$$

und/oder

$$\begin{cases} v_e \dfrac{dc_p}{dt} = \rho MBF(1 - HCT)c_{ap} + \rho\, PS\, c_e - \rho(MBF(1 - HCT) + PS)c_p \\ \qquad\qquad v_e \dfrac{dc_e}{dt} = \rho\, PS\, (c_p - c_e) \end{cases} ,$$

und/oder

$$\int_0^\infty TAC(t)dt = \int_0^\infty AIF(t) \otimes IRF(t)dt = \rho_t MBF \cdot \int_0^\infty AIF(t)dt \cdot MTT \ ,$$

wobei: $\tau$ - momentaner Zeitmoment im Beobachtungsintervall [0,t]; *AIF* - arterielle Eingabefunktion; *IRF* - Impulsantwortfunktion; *c* - Tracerkonzentration (*p* - Plasma, *e* - extrazellulär, extravaskulärer Raum, *ap* - arterielles Plasma); *v* - fraktionelles Plasmavolumen in einem Kompartiment; *PS* - Permeabilität-Oberflächenprodukt; t - Zeit *TAC* - Zeitabschwächungskurve; *AIF* - arterielle Eingabefunktion; *IRF* - Impulsantwortfunktion; *MBF* - myokardialer Blutfluss; und *MTT* - mittlere Transitzeit.

**14.** Das Verfahren nach einem der Ansprüche 1-13, wobei die Perfusionsmetriken beinhalten:

- myokardialer Blutfluss (MBF) definiert als das Blutvolumen, das mit einer bestimmten Geschwindigkeit durch das Myokardgewebe fließt,
- myokardiales Blutvolumen (MBV), das das Gesamtvolumen des Blutes in einem gegebenen Einheitsvolumen eines Myokards ist,
- mittlere Transitzeit (MTT), die der erwartete Wert des Zeitintervalls ist, das Blut innerhalb eines bestimmten Teils eines Myokards verbringt, und/oder
- Zeit bis zum Peak (TTP), die das Zeitintervall in der Radiodichte oder das Zeitintervall darstellt, in dem ein Indikator der Konzentration in einem bestimmten Teil eines Myokards sein Maximum erreicht, und

wobei die MBF, die MBV, die MTT und/oder die TTP als ein absoluter Wert oder als ein relativer Wert berechnet werden.

**15.** Computerlesbares Medium, das Anweisungen umfasst, die, wenn sie von einem Computer ausgeführt werden, den Computer veranlassen, die Schritte eines in einem der Ansprüche 1-14 definierten Verfahrens durchzuführen.

**Revendications**

**1.** Procédé mis en œuvre par ordinateur pour évaluer une perfusion myocardique en utilisant une mesure non invasive et une poromécanique pour un patient humain, le procédé comprenant :

- simuler (3) l'écoulement sanguin dans un modèle de géométrie anatomique ($\overline{\Omega}$) d'un myocarde lié à une pluralité de vaisseaux coronaires en utilisant mécanique des fluides numérique, la mécanique des fluides numérique comprenant au moins une condition aux limites spécifique au patient et au moins un paramètre physique pour décrire le sang en tant qu'un milieu d'écoulement, et

dans lequel le modèle de géométrie anatomique ($\overline{\Omega}$) d'un myocarde lié à une pluralité de vaisseaux coronaires comprend un domaine fluide ($\Omega_f$) et un domaine poreux ($\Omega_p$) et une interface ($\Gamma$) entre le domaine fluide ($\Omega_f$) et le domaine poreux ($\Omega_p$), le modèle de géométrie anatomique ($\overline{\Omega}$) d'un myocarde satisfaisant les équations suivantes : $\overline{\Omega} = \overline{\Omega_f} \cup \overline{\Omega_p}$ et $\Omega_f \cap \Omega_p = \emptyset$, et

dans lequel $\overline{\Omega_f}$ comprend ou consiste en le domaine fluide ($\Omega_f$) et une limite ($\Gamma_f$) du domaine fluide ($\Omega_f$) et $\overline{\Omega_p}$ comprend ou consiste en le domaine poreux ($\Omega_p$) et une limite ($\Gamma_p$) du domaine poreux ($\Omega_p$), et

dans lequel l'interface satisfait l'équation suivante: $\Gamma = \overline{\Omega_f} \cap \overline{\Omega_p} \neq \emptyset$, et

dans lequel ladite au moins une condition aux limites spécifique au patient est déterminée en utilisant des données de santé et démographiques spécifiques au patient et ledit au moins un paramètre physique pour décrire le sang en tant qu'un milieu d'écoulement est choisi en utilisant des données de santé et démographiques spécifiques au patient, et

dans lequel le modèle de géométrie anatomique ($\overline{\Omega}$) d'un myocarde lié à une pluralité de vaisseaux coronaires est construit en utilisant des données de structure anatomique spécifique au patient du patient humain et dans lequel des données de structure anatomique spécifique au patient du patient humain sont obtenues à partir d'au moins une ladite mesure non invasive,

dans lequel le domaine poreux ($\Omega_p$) est configuré pour décrire l'écoulement dans un tissu myocardique,

et dans lequel le procédé comprend en outre :

- calculer des mesures de perfusion en utilisant des résultats de la simulation d'écoulement sanguin dans le modèle de géométrie anatomique ($\overline{\Omega}$) d'un myocarde lié à une pluralité de vaisseaux coronaires.

2. Procédé selon la revendication 1, dans lequel on obtient des données de structure anatomique spécifiques au patient par au moins un parmi l'angiographie par tomodensitométrie, la résonance magnétique nucléaire ou l'angiographie assistée par ordinateur.

3. Procédé selon la revendication 2, dans lequel des données de structure anatomique spécifiques au patient comprennent des images stockées dans un format DICOM.

4. Procédé selon l'une quelconque des revendications 1-3, dans lequel la construction du modèle de géométrie anatomique ($\overline{\Omega}$) d'un myocarde lié à une pluralité de vaisseaux coronaires comprend la segmentation d'image comprise dans des données de structure anatomique spécifiques au patient pour aboutir à un modèle 3D surfacique des artères et des muscles du cœur du patient humain.

5. Procédé selon la revendication 4, dans lequel la construction (2.B) du modèle de géométrie anatomique ($\overline{\Omega}$) d'un myocarde lié à une pluralité de vaisseaux coronaires comprend en outre une étape, dans laquelle le modèle 3D surfacique est discrétisé par un mesh volumétrique non structuré.

6. Procédé selon l'une quelconque des revendications 1-5, dans lequel des données de santé et démographiques spécifiques au patient incluent l'âge, le sexe, la taille, le poids corporel, la pression sanguine générale, des résultats d'analyse de sang et/ou des renseignements sur le traitement pharmacologique actuel du patient, le traitement pharmacologique actuel du patient étant choisi parmi sans s'y limiter un bêta-bloquant, un inhibiteur de l'enzyme de conversion de l'angiotensine et/ou un agent antiarythmique.

7. Procédé selon l'une quelconque des revendications 1-6, dans lequel le paramètre physique est choisi dans le groupe comprenant la densité et un coefficient de viscosité dynamique.

8. Procédé selon l'une quelconque des revendications 1-7, dans lequel le domaine poreux ($\Omega_p$) est saturé.

9. Procédé selon l'une quelconque des revendications 1-8, dans lequel le domaine fluide ($\Omega_f$) satisfait l'équation suivante :

$$\begin{cases} \rho\left(\dfrac{\partial \mathbf{u}}{\partial t} + \mathbf{u} \otimes \nabla \mathbf{u}\right) - \eta \Delta \mathbf{u} + \nabla p = 0 \\ \nabla \mathbf{u} = 0 \end{cases}$$

et/ou dans lequel le domaine poreux ($\Omega_p$) satisfait l'équation suivante :

$$\nabla p = -\frac{\eta}{\mathbf{K}}\mathbf{u} - \frac{\rho C_F}{\sqrt{\mathbf{K}}}|\mathbf{u}|\mathbf{u}$$

et/ou dans lequel l'écoulement à travers le domaine fluide ($\Omega_f$) et le domaine poreux ($\Omega_p$) satisfait l'équation suivante :

$$\rho\left(\frac{\partial \mathbf{u}}{\partial t} + \mathbf{u} \otimes \nabla \mathbf{u}\right) - \eta \Delta \mathbf{u} + \nabla p + \chi\left(\frac{\eta}{\mathbf{K}}\mathbf{u} + \frac{\rho C_F}{\sqrt{\mathbf{K}}}|\mathbf{u}|\mathbf{u}\right) = 0 \ \text{ dans } \Omega_f \cap \Omega_p \ ,$$

où : $p$ - densité fluide ; $t$ - temps ; $\mathbf{u}$ et $p$ - vitesse et pression d'écoulement ; $\eta$ - coefficient de viscosité dynamique ; $\chi$ = 1 dans $\Omega_p$ ou $\chi$ = 0 hors de $\Omega_p$; $C_F$ - tenseur de résistance inertielle ; et $\mathbf{K}$ - tenseur de perméabilité.

10. Procédé selon l'une quelconque des revendications 1-9, dans lequel la mécanique des fluides numérique comprend des propriétés rhéologiques décrites en utilisant $(\eta - \eta_\infty) / (\eta_0 - \eta_\infty) = F(HCT, \dot{\gamma})$, où $\eta$ est le coefficient de viscosité dynamique et HCT est l'hématocrite.

11. Procédé selon la revendication 10, dans lequel $F(HCT, \dot{\gamma})$ est choisie selon le modèle de Cross et Carreau-Yasuda ou le modèle de Walburn et Schneck.

12. Procédé selon la revendication 1, dans lequel le calcul des mesures de perfusion comprend l'utilisation d'au moins une des méthodes de récupération cinétique de traceur régionale, dans lequel des méthodes de récupération cinétique de traceur régionale comprennent Pente MaXimale (MaXimum Slope, MXS), la déconvolution de la fonction de réponse d'impulsion (DRF), l'échange sang/tissu plurispécifique à voies multiples non linéaire (multi-pathway, multi-species and non-linear Blood-Tissue eXchange, BTX), et/ou dans lequel le calcul des mesures de perfusion comprend l'utilisation de méthode séquentielle de Monte Carlo pour la méthode Lagrangienne retraçant le trajectoire de ligne de courant sous la forme de

$$\boldsymbol{r}_n = \boldsymbol{r}_1 + \sum_{i=1}^{n-1} \Delta \boldsymbol{s}_i$$

où $\boldsymbol{s}_i$ sont des vecteurs du train $\{\Delta \boldsymbol{s}_1, \Delta \boldsymbol{s}_2, ..., \Delta \boldsymbol{s}_n\}$, où $\Delta \boldsymbol{s}_i$ = $f(t, \boldsymbol{r}_i)$.

13. Procédé selon la revendication 12, dans lequel des méthodes de récupération cinétique de traceur régionale comprend l'équation suivante :

$$TAC(t) = \int_0^t AIF(\tau) \cdot IRF(t - \tau) \cdot d\tau = AIF(t) \otimes IRF(t) \ ,$$

et/ou

$$\begin{cases} v_e \dfrac{dc_p}{dt} = \rho MBF(1 - HCT)c_{ap} + \rho\, PS\, c_e - \rho(MBF(1 - HCT) + PS)c_p \\ \qquad\qquad v_e \dfrac{dc_e}{dt} = \rho\, PS\left(c_p - c_e\right) \end{cases} ,$$

et/ou

$$\int_0^\infty TAC(t)dt = \int_0^\infty AIF(t) \otimes IRF(t)dt = \rho_t MBF \cdot \int_0^\infty AIF(t)dt \cdot MTT \, ,$$

où : $\tau$ - moment instantané pendant l'intervalle observationnel [0,t]; *AIF* - fonction d'entrée artérielle ; *IRF* - fonction de réponse d'impulsion ; *c* - concentration de traceur (*p*-plasma, e-espace extracellulaire, extravasculaire, *ap*-plasma artériel) ; *v* - volume plasmatique fractionnel dans un compartiment ; *PS* - produit de surface de perméabilité ; *t* - temps ; *TAC* - courbe d'atténuation temporelle ; *AIF* - fonction d'entrée artérielle ; *IRF* - fonction de réponse d'impulsion ; *MBF* - l'écoulement sanguin myocardique.

14. Procédé selon l'une quelconque des revendications 1-13, dans lequel des mesures de perfusion incluent :

   • l'écoulement sanguin myocardique (MBF) défini en tant que le volume du sang transitant à travers du tissu myocardique à un débit donné ;
   • le volume du sang myocardique (MBV) est le volume total du sang dans un volume d'unité donné d'un myocarde ;
   • le temps du transit moyen (MTT) est l'espérance mathématique d'intervalle temporel que le sang séjourne dans une partie particulière d'un myocarde, et/ou
   • le temps de montée (TTP) représente l'intervalle temporel en radiodensité ou l'intervalle temporel dans lequel un indicateur de concentration sera à son maximum dans une partie particulière d'un myocarde, et

   dans lequel le MBF, le MBV, le MTT et/ou le TTP sont calculés en tant qu'une valeur absolue ou une valeur relative.

15. Support de stockage lisible par ordinateur comprenant des instructions qui, lorsqu'elles sont exécutées par un ordinateur, amènent l'ordinateur à exécuter les étapes du procédé tel que défini dans l'une quelconque des revendications 1-14.

FIG. 1

**(A) arterial input / tissue attenuation**

$$AIF(t) = \frac{4384.6}{\Gamma(8.175) \cdot 1} \cdot \left(\frac{t - 2.655}{1}\right)^{7.175} \cdot \exp\left(-\frac{t - 2.655}{1}\right)$$

**(B)**

FIG. 2

FIG. 3

FIG. 4

FIG. 5

(A) in vivo results

MBF [ml/100ml/min]

(B) computational fluid dynamics results

Normalized flow rate

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019183579 A1

**Non-patent literature cited in the description**

- **VILLARROEL MA** ; **BLACKWELL DL** ; **JEN A**. Tables of Summary Health Statistics for U.S. Adults: 2018 National Health Interview Survey. National Center for Health Statistics, 15 December 2021
- **BARRETT KE**. Ganong's Review of Medical Physiology. McGraw-Hill Education, 2019
- **ARGUETA EE** ; **PANIAGUA D**. *Thermodilution cardiac output, Cardiology in Review*, 2019, vol. 27 (3), 138-144
- **KENNETH ZIERLER** ; **MEIER P** ; **ZIERLER KL**. On the theory of the indicator-dilution method for measurement of blood flow and volume. *The Journal of Applied Physiology*, 1954, vol. 6 (12), 731-744
- **ZIERLER K**. Indicator dilution methods for measuring blood flow, volume, and other properties of biological systems: a brief history and memoir. *Annals of Biomedical Engineering*, 2000, vol. 28 (8), 836-848
- **TAYLOR SH**. Measurement of the cardiac output in man. *Proc R Soc Med, 59 Suppl(Suppl 1)*, 1966, 35-53
- **AXEL L**. Cerebral blood flow determination by rapid-sequence computed tomography: theoretical analysis. *Radiology*, 1980, vol. 137 (3), 679-86
- **LEE T-Y**. *Functional CT: Physiological models, Trends Biotechnol*, 2002, vol. 20, S3-S10
- **TOMANDL BF et al.** *Comprehensive imaging of ischemic stroke with multisection CT, Radiographics*, 2003, vol. 23 (3), 565-92
- **KONSTAS AA et al.** Theoretic basis and technical implementations of CT perfusion in acute ischemic stroke. Part 1: Theoretic basis. *AJNR Am J Neuroradiol*, 2009, vol. 30 (4), 662-8
- **ALJAROUDI WA** ; **HAGE FG**. Review of cardiovascular imaging in the Journal of Nuclear Cardiology 2019: Positron emission tomography, computed tomography and magnetic resonance. *J Nucl Cardiol*, 2020, vol. 27 (3), 921-930
- **DEWEY M**. Clinical quantitative cardiac imaging for the assessment of myocardial ischaemia. *Nature Reviews Cardiology*, 2020, vol. 17 (7), 427-450

- **KITAGAWA K**. Dynamic CT perfusion imaging: state of the art. *Cardiovascular Imaging Asia*, 2018, vol. 2 (2), 38-48
- **XU C** ; **YI Y** ; **WANG Y**. Clinical Applications of CT Myocardial Perfusion Imaging. *Cardiovascular Imaging Asia*, 2020, vol. 4 (4), 86-90
- **SEITUN S**. CT myocardial perfusion imaging: a new frontier in cardiac imaging. *BioMed research international*, 2018
- **SEITUN S**. Stress computed tomography myocardial perfusion imaging: a new topic in cardiology. *Revista Española de Cardiologia*, 2016, vol. 69 (2), 188-200
- **KITAGAWA K**. Dynamic CT perfusion imaging: State of the art. *Cardiovasc Imaging Asia*, 2018, vol. 2 (2), 38-48
- **SEITUN S**. CT myocardial perfusion imaging: a new frontier in cardiac imaging. *BioMed research international*, 2018, vol. 7295460, 21
- **DANAD I**. Static and dynamic assessment of myocardial perfusion by computed tomography. *European Heart Journal-Cardiovascular Imaging*, 2016, vol. 17 (8), 836-844
- **FLORKOWSKI T** ; **DAVIS TG**. The measurement of high discharges in turbulent rivers using tritium tracer. *Journal of Hydrology*, 1969, vol. 8 (3), 249-264
- **KILPATRICK FA** ; **COBB ED**. Measurement of discharge using tracers. U.S. Department of the Interior, U.S. Geological Survey, 1982
- **EVANS GV**. Tracer techniques in hydrology. *The International Journal of Applied Radiation and Isotopes*, 1983, vol. 34 (1), 451-475
- **ALLEN CM**. Hydraulic - turbine tests by the Allen method. *Power Plant Engineering*, 1927, vol. 31 (10), 549-551
- **ASSAAD FA** ; **LAMOREAUX PE** ; **HUGHES T**. Field methods for geologists and hydrogeologists. Springer-Verlag Berlin and Heidelberg GmbH & Co., 2012
- **WINGARD LB et al.** Concepts of residence time distribution applied to the indicator-dilution method. *J Appl Physiol*, 1972, vol. 33 (2), 264-275

- **CHEN K**. CFD simulation of particle residence time distribution in industrial scale horizontal fluidized bed. *Powder Technology*, 2018, vol. 345 (1), 129-139
- **WALKER P** ; **SHEIKHOLESLAMI R**. Assessment of the effect of velocity and residence time in CaSO4 precipitating flow reaction. *Chemical Engineering Science*, 2003, vol. 58 (16), 3807-3816
- Effect of porous media of the stenosed artery wall to the coronary physiological diagnostic parameter: A computational fluid dynamic analysis. **G. KALIMUTHU et al.** Atherosclerosis. Elsevier, 31 January 2014, vol. 233, 630-635
- Critical assessment of arterial transport models. **KHAKPOUR et al.** International Journal of Heat and Mass Transfer. Elsevier, 28 June 2007, vol. 51, 807-822
- Non-Newtonian models for molecular viscosity and wall shear stress in a 3D reconstructed human left coronary artery. **SOULIS et al.** Medical Engineering & Physics. Butterworth-Heinemann, 31 December 2007, vol. 30, 9-19
- CFD Generated Tracer Indicator Flow Assessment using Perfusion MRI Analysis. IEEE-EMBS Conference on Biomedical Engineering and Science (IECBES). IEEE, 01 March 2021, 51-56
- In silico coronary wave intensity analysis: application of an integrated one-dimensional and poromechanical model of cardiac perfusion. *Biomech Model Mechanobiol*, vol. 15 (6), 1535-1555
- *J Nucl Med.*, vol. 34 (4), 706-13
- **KLOSKA SP et al.** Increasing sampling interval in cerebral perfusion CT: limitation for the maximum slope model. *Acad Radiol*, 2010, vol. 17 (1), 61-66
- **WIRESTAM R**. Using contrast agents to obtain maps of regional perfusion and capillary wall permeability. *Imaging in Medicine*, 2012, vol. 4 (4), 423-438
- **BINDSCHADLER M et al.** Comparison of blood flow models and acquisitions for quantitative myocardial perfusion estimation from dynamic CT. *Phys Med Biol*, 2014, vol. 59 (7), 1533-56
- **TOFTS PS et al.** Estimating kinetic parameters from dynamic contrast-enhanced T(1)-weighted MRI of a diffusable tracer: standardized quantities and symbols. *J Magn Reson Imaging*, 1999, vol. 10 (3), 223-32
- **LIU P et al.** Perfusion imaging: An advection diffusion Approach. *IEEE Transactions on Medical Imaging*, 2021, vol. 40 (12), 3424-3435
- **BIRD RB et al.** Transport phenomena. John Wiley & Sons, 2006
- Transport phenomena in porous media, Vafai K. **INGHAM D** ; **POP I**. Handbook of porous media. CRC Press, 2005
- **ALVES JR et al.** Simulation of the perfusion of contrast agent used in cardiac magnetic resonance: a step toward non-invasive cardiac perfusion quantification. *Front Physiol*, 2019, vol. 10, 177
- **ALVES JR et al.** Simulation of cardiac perfusion by contrast in the myocardium using a formulation of flow in porous media. *J. Comput. Appl. Math*, 2016, vol. 295 (C), 13-24
- **VLADIMIR LUKES et al.** Numerical simulation of liver perfusion: from CT scans to FE model. *CoRR, abs/1412.6412*, 2014
- **PAPAMANOLIS L et al.** Myocardial perfusion simulation for coronary artery disease: a coupled patient-specific multiscale model. *Ann Biomed Eng*, 2021, vol. 49 (5), 1432-1447
- Toward myocardium perfusion from x-ray computed tomography. **JAQUET C**. Ph.D. Thesis. Université Paris Est, 2018
- **WYLIE EB** ; **STREETER VL**. Hydraulics Transients. McGraw-Hill, 1967
- **SHARMA P et al.** A patient-specific reduced-order model for coronary circulation. *2012 9th IEEE International Symposium on Biomedical Imaging*, 2012, 832-835
- **FORMAGGIA L et al.** One-dimensional models for blood flow in arteries. *Journal of Engineering Mathematics*, 2003, vol. 47, 251-276
- **POZRIKIDIS C**. Introduction to theoretical and computational fluid dynamics. Oxford University Press, 2011
- **CHUNG TJ**. Computational Fluid Dynamics Cambridge. University Press, 2010
- **DE GRUTTOLA S et al.** Computational simulation of a non-newtonian model of the blood separation process. *Artif Organs*, 2005, vol. 29 (12), 949-59
- **MILNOR WR**. Hemodynamics. Williams&Wilkins, 1982
- **VLACHOPOULOS CH et al.** McDonald's blood flow in arteries. CRC Press, 2011
- **KANNOJIYA V et al.** Simulation of Blood as Fluid: A Review from Rheological Aspects. *IEEE Rev Biomed Eng*, 2021, vol. 14, 327-341
- **SAMSEL RW** ; **PERELSON AS**. Kinetics of rouleau formation. I. A mass action approach with geometric features. *Biophys J*, 1982, vol. 37 (2), 493-514
- **SAMSEL RW** ; **PERELSON AS**. Kinetics of rouleau formation. II. Reversible reactions. *Biophys J*, 1984, vol. 45 (4), 805-2
- **CARREAU PJ**. Rheological equations from molecular network theories. *Journal of Rheology*, 1972, vol. 16 (1), 99-127
- **CROSS MM**. Rheology of non-Newtonian fluids: A new flow equation for pseudoplastic system. *Journal of Colloid Science*, 1965, vol. 20 (5), 417-437

- Investigation of the analogies between viscometric and linear viscoelastic properties of polystyrene fluids. **YASUDA K**. Ph.D. Thesis. Massachusetts Institute of Technology, 1979
- **WALBURN FJ** ; **SCHNECK D**. A constitutive equation for whole human blood. *Biorheology*, 1976, vol. 1, 201-210
- **FORCHHEIMER P**. Wasserbewegung durch Boden. *Z. Ver. Deutsch. Ing*, 1901, vol. 45, 1782-1788
- **WHITAKER S**. The Forchheimer equation: A theoretical development. *Transport in Porous Media*, 1996, vol. 25, 2761
- **VAFAI K**. Handbook of porous media. CRC Press, 2015
- **BRUNEAU CH** ; **MORTAZAVI I**. Numerical modelling and passive flow control using porous media. *Computers & Fluids*, 2008, vol. 37 (5), 488-498
- **CRANE M** ; **BLUNT M**. Streamline-based simulation of solute transport. *Water Resour. Res*, 1999, vol. 35, 3061-3078
- **SALAMON P et al.** A review and numerical assessment of the random walk particle tracking method. *J. Contam. Hydrol*, 2006, vol. 87, 277-305
- **FRIMAN O et al.** Probabilistic 4D blood flow mapping. *International Conference on Medical Image Computing and Computer-Assisted Intervention, 13(Pt 3)*, 2010, 416-23
- **BEAR J**. Modeling phenomena of flow and transport in porous media. Springer International, 2018
- **VINNAKOTA KC** ; **BASSINGTHWAIGHTE JB**. Myocardial density and composition: a basis for calculating intracellular metabolite concentrations. *American Journal of Physiology-Heart and Circulatory Physiology*, 2004, vol. 286 (5), H1742-H1749
- **GHEORGHE AG et al.** Cardiac left ventricular myocardial tissue density, evaluated by computed tomography and autopsy. *BMC Medical Imaging*, 2019, vol. 19, 29
- **CUONG NLQ et al.** Porosity estimation from high resolution CT SAN images of rock samples by using Housfield unit. *Open Journal of Geology*, 2018, vol. 8, 1019-1026
- **SUDHYADHOM A**. On the molecular relationship between Hounsfield Unit (HU), mass density, and electron density in computed tomography (CT). *PLoS ONE*, 2020, vol. 15 (12), e024486
- **CERQUEIRA MD**. Standardized myocardial segmentation and nomenclature for tomographic imaging of the heart. A statement for healthcare professionals from the Cardiac Imaging Committee of the Council on Clinical Cardiology of the American Heart Association. *Circulation*, 2002, vol. 105 (4), 539-42
- **KONO AK et al.** Relative myocardial blood flow by dynamic computed tomographic perfusion imaging predicts hemodynamic significance of coronary stenosis better than absolute blood flow. *Invest Radiol*, 2014, vol. 49 (12), 801-7
- **COENEN A et al.** Integrating CT myocardial perfusion and CT-FFR in the work-up of coronary artery disease. *JACC Cardiovasc Imaging*, 2017, vol. 10 (7), 760-770